# EUROPEAN PATENT APPLICATION

(11) **EP 4 600 350 A1**
(43) Date of publication of application: **13.08.2025**
(21) Application number: 23874802.4
(22) Date of filing: 02.10.2023
(51) Int. Cl.: C12N 5/0775, A61K 35/32, A61P 1/04, A61P 9/10, A61P 25/00, A61P 29/00, A61P 37/06

(54) **NOVEL DENTAL PULP STEM CELL POPULATION**

(30) Priority: 03.10.2022 WO PCT/JP2022/036892; 15.05.2023 JP 2023080181
(71) Applicant: Kidswell Bio Corporation, Tokyo 104-0033 (JP)
(72) Inventor: MITANI Yasuyuki, Tokyo 104-0033 (JP); ARIKI Hiromi, Sapporo-shi, Hokkaido 001-0021 (JP); FUKUDA Noritaka, Sapporo-shi, Hokkaido 001-0021 (JP)
(74) Representative: Hoffmann Eitle
(86) International application number: PCT/JP2023/035858
(87) International publication number: WO 2024/075675

(57) **Abstract**

The present invention relates, for example, to a stem cell population derived from human deciduous dental pulp, the stem cell population being characterized in that 90% or more of the stem cell population is CD117-negative, CD73-positive, CD90-positive, and CD105-positive, and to a method for producing a stem cell population derived from human deciduous dental pulp, comprising the step of culturing cells isolated from the human deciduous dental pulp in a medium free of FBS (fetal bovine serum) in the presence of a human platelet lysate (hPL).

## Description

### [Technical Field]

### Related application:

The present specification incorporates the contents described in the specifications of PCT/JP2022/036892 (filed on October 3, 2022) and Japanese Patent Application No. 2023-080181 (filed on May 15, 2023) on which the priority of the present application is based.

### Technical Field:

The present invention relates to a stem cell population derived from human deciduous dental pulp and a culture supernatant thereof, a pharmaceutical composition comprising the stem cell population, and a method for producing the same.

### [Background Art]

Stem cells derived from human dental pulp (dental pulp stem cells) have been reported to have a therapeutic effect on spinal cord injury, cerebral palsy (perinatal hypoxic ischemic encephalopathy), lower limb ischemia, and the like in experiments using animal models.
However, according to the previous reports, the cells were administered directly to the spinal cord immediately after spinal cord injury (Non Patent Literature 1) or administered directly to the brain several hours after brain injury in cerebral palsy models (Non Patent Literature 2). These approaches are not realistic administration methods when clinical application is taken into consideration. In other cerebral palsy models, the cells were intravenously administered 24 hours after brain injury, though improvement in motor function was observed more than 5 months later (Non Patent Literature 3). Thus, there is a demand for a treatment method that produces an improvement effect earlier. As for lower limb ischemia, the cells were administered to model animals with moderate symptoms immediately after vascular ligature (Non Patent Literature 4) or several hours thereafter (Non Patent Literature 5). These approaches are detached from the actual status of treatment of severe lower limb ischemia mainly targeted by stem cell therapy.

Fetal bovine serum (FBS) has been used in methods for isolating dental pulp stem cells from dental pulp tissues (Non Patent Literatures 1 to 6). However, the possibility cannot be completely excluded that a foreign serum component remains in administered preparations. Therefore, use of FBS is not favorable when clinical application is taken into consideration. In the case of using cells isolated from dental pulp tissues of patients themselves, the cells may be prepared using the autologous serum of the patients. However, problems are burden on the patients as well as increase in time and cost required for preparing the cells using the autologous serum on individual-basis. For rendering treatment with dental pulp stem cells available to more patients, it is desired to produce allogeneic cells in a large amount and use the cells as an off-the-shelf product to the treatment.

Dental pulp stem cells isolated by the conventional methods using FBS have been analyzed for their cellular characteristics. For example, characteristics have been reported in which the cell population comprises 50% or more or 96.2% CD117 (c-kit)-positive cells (Patent Literature 1 and Non Patent Literature 7). In preceding report, CD117-positive cells have been separated and reported to have multipotency as their characteristics (Non Patent Literature 8). In addition, the dental pulp stem cells have been reported to be CD325 (N-cadherin)-positive (Non Patent Literature 9) and to include both cells positive and negative for CD51 (integrin αV) and CD49d (integrin α4) (Non Patent Literatures 10 and 11).

There is a report stating that multipotent stem cell population is obtained by treating a human dental pulp tissue with trypsin and then culturing the tissue in a serum-free medium containing a human platelet lysate (hPL). However, the obtained cell population is positive for both of hematopoietic stem cell markers CD34 and CD45 and is therefore considered as a heterogeneous cell population (Non Patent Literature 12).

### [Citation List]

### [Patent Literature]

[Patent Literature 1] WO2014/141210A2 (JP6491606B)

### [Non Patent Literature]

[Non Patent Literature 1] Sakai et al., J Clin Invest. 2012 Jan; 122 (1): 80-90. doi: 10.1172/JCI59251.
[Non Patent Literature 2] Yamagata et al., Stroke. 2013 Feb; 44 (2): 551-4. doi: 10.1161/STROKEAHA.112.676759.
[Non Patent Literature 3] Kitase et al., Stem Cells Dev. 2020 Jan 15; 29 (2): 63-74. doi: 10.1089/scd.2019.0221. PMID: 31801412.
[Non Patent Literature 4] Yong et al., Tissue Eng Regen Med. 2022 Aug; 19 (4): 861-870. doi: 10.1007/s13770-022-00452-6.
[Non Patent Literature 5] Li et al., Stem Cells Int. 2021 Aug 31; 2021: 5585255. doi: 10.1155/2021/5585255.
[Non Patent Literature 6] Miura et al., Proc Natl Acad Sci U S A. 2003 May 13; 100 (10): 5807-12. doi: 10.1073/pnas.0937635100.
[Non Patent Literature 7] Suchanek et al,. Acta Medica (Hradec Kralove). 2010; 53 (2): 93-9. doi: 10.14712/18059694.2016.66.
[Non Patent Literature 8] Carnevale et al., J Tissue Eng Regen Med. 2018 Feb; 12 (2): e774-e785. doi: 10.1002/term.2378.
[Non Patent Literature 9] Takahashi et al., Arch Oral Biol. 2012 Jan; 57 (1): 44-51. doi: 10.1016/j.archoralbio.2011.07.013.
[Non Patent Literature 10] Lee et al., Stem Cell Res Ther. 2020 Jun 3; 11 (1): 210. doi: 10.1186/s13287-020-01714-7.
[Non Patent Literature 11] Hata et al., Stem Cell Res Ther. 2015 Sep 7; 6 (1): 162. doi: 10.1186/s13287-015-0156-4.
[Non Patent Literature 12] Pilbauerova et al., Biomolecules. 2022 Aug 8; 12 (8): 1091. doi: 10.3390/biom12081091.

### [Summary of Invention]

### [Technical Problem]

An object of the present invention is to provide safe and highly functional stem cells suitable for clinical application, and a method for producing the same.

### [Solution to Problem]

The inventors have found that a stem cell population obtained by culturing cells isolated from human deciduous dental pulp in a medium containing a human platelet lysate (hPL) has a structural feature (surface marker expression) different from that of previously reported dental pulp stem cells. The inventors have further found that the stem cell population obtained by this method has a higher amount of production of a cytokine useful for tissue regeneration than that of human deciduous tooth-derived dental pulp stem cells obtained by conventional methods using FBS and is capable of exerting excellent tissue regeneration capacity.

The present invention is based on these findings and provides the following [1] to [30] according to the first embodiment.
[1] A (non-transgenic) stem cell population derived from human dental pulp, the stem cell population being characterized in that 90% or more of the stem cell population is CD117-negative, CD73-positive, CD90-positive, and CD105-positive, wherein the human dental pulp is human deciduous dental pulp.
[2] The stem cell population according to [1], wherein 90% or more of the stem cell population is CD325-negative or is CD51-positive; preferably, 90% or more of the stem cell population is CD325-negative and is CD49d-positive and/or CD51-positive; more preferably, 90%, 95%, 97%, 98% or 99% or more of the stem cell population is CD325-negative and is CD49d-positive and/or CD51-positive.
[3] The stem cell population according to [1] or [2], wherein the stem cell population is CD31-negative.
[4] The stem cell population according to any of [1] to [3], wherein the stem cell population is CD34-negative and CD45-negative.
[5] The stem cell population according to any of [1] to [4], wherein the stem cell population produces SCF (stem cell factor) at a weight ratio of 1/20 or more, preferably 1/10 or more, relative to IL-6.
[6] The stem cell population according to any of [1] to [5], wherein the stem cell population produces SCF (stem cell factor) at a weight ratio of 1/10 to 1/2, preferably 1/10 to 1/3, relative to IL-6.
[7] The stem cell population according to any of [1] to [6], wherein the stem cell population produces at least 0.1 ng, 0.13 ng, or 0.15 ng, preferably 0.17 ng, more preferably 0.20 ng, of SCF in 48 hours per 1 × 10⁶ cells.
[8] The stem cell population according to any of [1] to [7], wherein the stem cell population produces ANGPTL4 (angiopoietin-like 4) at a weight ratio of 3 or more, preferably 5 or more, relative to IL-6.
[9] The stem cell population according to any of [1] to [8], wherein the stem cell population produces ANGPTL4 (angiopoietin-like 4) at a weight ratio of 5 to 20, preferably 5 to 15, relative to IL-6.
[10] The stem cell population according to any of [1] to [9], wherein the stem cell population produces at least 4.0 ng, 4.5 ng, or 5.0 ng, preferably 6.9 ng, more preferably 7.0 ng, of ANGPTL4 in 48 hours per 1 × 10⁶ cells.
[11] The stem cell population according to any of [1] to [10], wherein the stem cell population produces BIGH3 (transforming growth factor-beta-induced) at a weight ratio of 400 or more, preferably 450 or more, relative to IL-6.
[12] The stem cell population according to any of [1] to [11], wherein the stem cell population produces BIGH3 (transforming growth factor-beta-induced) at a weight ratio of 450 to 1500, preferably 450 to 1000, relative to IL-6.
[13] The stem cell population according to any of [1] to [12], wherein the stem cell population produces at least 400 ng, 450 ng, or 500 ng, preferably 550 ng, more preferably 600 ng, of BIGH3 in 48 hours per 1 × 10⁶ cells.
[14] The stem cell population according to any of [1] to [13], wherein the stem cell population is obtained by culturing cells enzymatically (e.g., using an enzyme containing collagenase, or an enzyme containing collagenase and neutral protease (dispase or thermolysin)) isolated from the human dental pulp in a medium free of FBS (fetal bovine serum) in the presence of a human platelet lysate (hPL).
[15] The stem cell population according to [14], wherein the medium is a serum-free medium.
[16] A culture supernatant of the stem cell population according to any of [1] to [15], the culture supernatant having at least one of the following characteristics 1) to 3):
   1) comprising SCF (stem cell factor) at a weight ratio of 1/20 or more, preferably 1/10 or more, relative to IL-6,
   2) comprising ANGPTL4 (angiopoietin-like 4) at a weight ratio of 3 or more, preferably 5 or more, relative to IL-6,
   3) comprising BIGH3 (transforming growth factor-beta-induced) at a weight ratio of 400 or more, preferably 450 or more, relative to IL-6.
[17] A culture supernatant of the stem cell population according to any of [1] to [15], the culture supernatant having at least one of the following characteristics 1) to 3):
   1) comprising SCF (stem cell factor) at a weight ratio of 1/10 to 1/2, preferably 1/10 to 1/3, relative to IL-6,
   2) comprising ANGPTL4 (angiopoietin-like 4) at a weight ratio of 5 to 20, preferably 5 to 15, relative to IL-6,
   3) comprising BIGH3 (transforming growth factor-beta-induced) at a weight ratio of 450 to 1500, preferably 450 to 1000, relative to IL-6.
[18] A pharmaceutical composition according to the following [18-1] or [18-2].
   [18-1] A pharmaceutical composition comprising the stem cell population according to any of [1] to [15], wherein the stem cell population has at least one of the following characteristics 1) to 3):
      1) producing SCF (stem cell factor) at a weight ratio of 1/20 or more, preferably 1/10 or more, relative to IL-6,
      2) producing ANGPTL4 (angiopoietin-like 4) at a weight ratio of 3 or more, preferably 5 or more, relative to IL-6,
      3) producing BIGH3 (transforming growth factor-beta-induced) at a weight ratio of 400 or more, preferably 450 or more, relative to IL-6.
   [18-2] A pharmaceutical composition comprising a culture supernatant of the stem cell population according to any of [1] to [15], wherein the pharmaceutical composition has at least one of the following characteristics 1) to 3):
      1) comprising SCF (stem cell factor) at a weight ratio of 1/20 or more, preferably 1/10 or more, relative to IL-6,
      2) comprising ANGPTL4 (angiopoietin-like 4) at a weight ratio of 3 or more, preferably 5 or more, relative to IL-6,
      3) comprising BIGH3 (transforming growth factor-beta-induced) at a weight ratio of 400 or more, preferably 450 or more, relative to IL-6.
[19] A pharmaceutical composition according to the following [19-1] or [19-2].
   [19-1] A pharmaceutical composition comprising the stem cell population according to any of [1] to [15], wherein the stem cell population has at least one of the following characteristics 1) to 3):
      1) producing SCF (stem cell factor) at a weight ratio of 1/10 to 1/2, preferably 1/10 to 1/3, relative to IL-6,
      2) producing ANGPTL4 (angiopoietin-like 4) at a weight ratio of 5 to 20, preferably 5 to 15, relative to IL-6,
      3) producing BIGH3 (transforming growth factor-beta-induced) at a weight ratio of 450 to 1500, further preferably 450 to 1000, relative to IL-6.
   [19-2] A pharmaceutical composition comprising a culture supernatant of the stem cell population according to any of [1] to [15], wherein the pharmaceutical composition has at least one of the following characteristics 1) to 3):
      1) comprising SCF (stem cell factor) at a weight ratio of 1/10 to 1/2, preferably 1/10 to 1/3, relative to IL-6,
      2) comprising ANGPTL4 (angiopoietin-like 4) at a weight ratio of 5 to 20, preferably 5 to 15, relative to IL-6,
      3) comprising BIGH3 (transforming growth factor-beta-induced) at a weight ratio of 450 to 1500, preferably 450 to 1000, relative to IL-6.
[20] The pharmaceutical composition according to [18] or [19] for treating or preventing any disease selected from spinal cord injury (including chronic-phase spinal cord injury), ischemic disease (cerebral infarction, lower limb ischemia, etc.), inflammatory disease, autoimmune disease, neurodegenerative disease, peripheral nerve disease, intestinal disease, and bone disease.
[21] A method for producing a stem cell population derived from human dental pulp, the method comprising the steps of:
   enzymatically (e.g., using an enzyme containing collagenase, or an enzyme containing collagenase and neutral protease (dispase or thermolysin)) isolating cells from the human dental pulp; culturing the cells in a medium free of FBS (fetal bovine serum) in the presence of a human platelet lysate (hPL); and obtaining a colony-forming cell population as the stem cell population. In the method, the cells may be subcultured (expansion-cultured), if necessary.
[22] The method according to [21], wherein the medium is a serum-free medium.
[23] The method according to [21] or [22], wherein the medium is animal-free.
[24] The method according to any of [21] to [23], wherein the obtained dental pulp stem cell population is CD117-negative.
[25] The method according to any of [21] to [24], wherein the obtained dental pulp stem cell population further has the characteristic(s) of the stem cell population according to any of [1] to [13].
[26] The method according to any of [21] to [24], wherein the human dental pulp is dental pulp of a deciduous tooth within 48 hours after tooth extraction.
[27] The stem cell population according to any of [1] to [15], wherein the human dental pulp is dental pulp of a deciduous tooth within 48 hours after tooth extraction.
[28] The stem cell population according to any of [1] to [15], wherein the stem cell population has the ability to differentiate into adipocytes, osteoblasts, chondrocytes, and neurons.
[29] The stem cell population according to any of [1] to [15], the culture supernatant according to [16] or [17], or the pharmaceutical composition according to any of [18] to [20], wherein the stem cell population, the culture supernatant, or the pharmaceutical composition has at least one effect selected from a neural progenitor cell proliferative effect, a neurite elongating effect, a vascular endothelial cell proliferative effect, a vascular endothelial cell recruiting effect, a vascular-like structure constructing effect, and an immunosuppressive effect.
[30] The method according to any of [21] to [23], wherein the medium comprises a 10% human platelet lysate.

In [1] to [29] described above, the terms "positive" and "negative" have meanings as defined in the present specification.

The present invention provides the following [1] to [20] according to the second embodiment.
[1] A method for treating a disease in need of tissue regeneration, the method comprising administering a (non-transgenic) stem cell population derived from human dental pulp and/or a culture supernatant of the stem cell population to a subject in need thereof, the stem cell population being characterized in that 90% or more of the stem cell population is CD117-negative, CD73-positive, CD90-positive, and CD105-positive, wherein the human dental pulp is human deciduous dental pulp.
[2] The method according to [1], wherein 90% or more of the stem cell population is CD325-negative or is CD51-positive; preferably, 90% or more of the stem cell population is CD325-negative and is CD49d-positive and/or CD51-positive; more preferably, 90%, 95%, 97%, 98% or 99% or more of the stem cell population is CD325-negative and is CD49d-positive and/or CD51-positive.
[3] The method according to [1], wherein the stem cell population is CD31-negative.
[4] The method according to [1], wherein the stem cell population is CD34-negative and CD45-negative.
[5] The method according to [1], wherein the stem cell population produces SCF (stem cell factor) at a weight ratio of 1/10 or more relative to IL-6.
[6] The method according to [1], wherein the stem cell population produces SCF (stem cell factor) at a weight ratio of 1/10 to 1/3 relative to IL-6.
[7] The method according to [1], wherein the stem cell population produces at least 0.1 ng of SCF in 48 hours per 1 × 10⁶ cells.
[8] The method according to [1], wherein the stem cell population produces ANGPTL4 (angiopoietin-like 4) at a weight ratio of 5 or more relative to IL-6.
[9] The method according to [1], wherein the stem cell population produces ANGPTL4 (angiopoietin-like 4) at a weight ratio of 5 to 20 relative to IL-6.
[10] The method according to [1], wherein the stem cell population produces at least 5 ng of ANGPTL4 in 48 hours per 1 × 10⁶ cells.
[11] The method according to [1], wherein the stem cell population produces BIGH3 (transforming growth factor-beta-induced) at a weight ratio of 450 or more relative to IL-6.
[12] The method according to [1], wherein the stem cell population produces BIGH3 (transforming growth factor-beta-induced) at a weight ratio of 450 to 1000 relative to IL-6.
[13] The method according to [1], wherein the stem cell population produces at least 500 ng of BIGH3 in 48 hours per 1 × 10⁶ cells.
[14] The method according to [1], wherein the stem cell population is obtained by culturing cells enzymatically isolated from the human dental pulp in a medium free of FBS (fetal bovine serum) in the presence of a human platelet lysate (hPL).
[15] The method according to [14], wherein the medium is a serum-free medium.
[16] The method according to [14], wherein the medium is animal-free.
[17] The method according to [1], wherein the disease in need of tissue regeneration is any disease selected from spinal cord injury (including chronic-phase spinal cord injury), ischemic disease, inflammatory disease, autoimmune disease, and intestinal disease.
[18] The method according to [1], wherein the culture supernatant of the stem cell population has at least one of the following characteristics 1) to 3):
   1) comprising SCF (stem cell factor) at a weight ratio of 1/10 or more relative to IL-6,
   2) comprising ANGPTL4 (angiopoietin-like 4) at a weight ratio of 5 or more relative to IL-6,
   3) comprising BIGH3 (transforming growth factor-beta-induced) at a weight ratio of 450 or more relative to IL-6.
[19] The method according to [1], wherein the culture supernatant of the stem cell population has at least one of the following characteristics 1) to 3):
   1) comprising SCF (stem cell factor) at a weight ratio of 1/10 to 1/3 relative to IL-6,
   2) comprising ANGPTL4 (angiopoietin-like 4) at a weight ratio of 5 to 20 relative to IL-6,
   3) comprising BIGH3 (transforming growth factor-beta-induced) at a weight ratio of 450 to 1000 relative to IL-6.
[20] The method according to [1], wherein the human dental pulp is dental pulp of a deciduous tooth within 48 hours after tooth extraction.

### [Advantageous Effects of Invention]

The present invention provides a highly functional and safe dental pulp stem cell population. Unlike conventional methods, the stem cell population of the present invention is prepared using a serum-free medium containing no Xenogeneic component such as FBS and obtained as a highly homogeneous cell population without any special separation approach.

### [Brief Description of Drawings]

[Figure 1] Figure 1 shows surface marker analysis results of the human dental pulp stem cells according to the present invention. Figure 1A: CD31, Figure 1B: CD34
[Figure 2] Figure 2 shows a microscopic image obtained by the oil red staining of adipocytes (fat droplets) induced by differentiation from the human dental pulp stem cells.
[Figure 3] Figure 3 shows a microscopic image obtained by the calcification staining of osteoblasts induced by differentiation from the human dental pulp stem cells.
[Figure 4] Figure 4 shows a microscopic image obtained by the alcian blue staining of a cartilage tissue induced by differentiation from the human dental pulp stem cells.
[Figure 5] Figure 5 shows immunostaining images of neurons induced by differentiation from the human dental pulp stem cells (isotype control, anti-Nestin, anti-βIII-tubulin, anti-neurofilament M immunostaining images in order from the left).
[Figure 6] Figure 6 shows a neurite elongating effect of a culture supernatant of the human dental pulp stem cells.
[Figure 7] Figure 7 shows a neural progenitor cell proliferative effect of a culture supernatant of the human dental pulp stem cells.

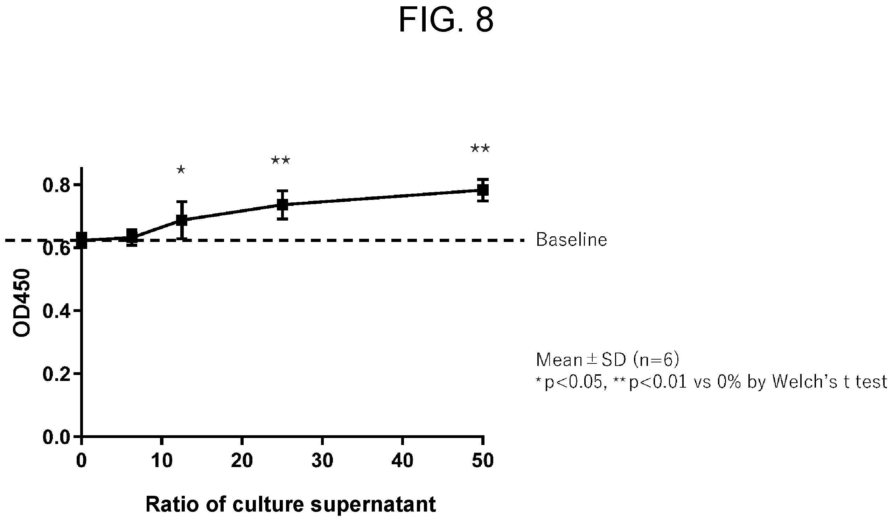

[Figure 8] Figure 8 shows a vascular endothelial cell proliferative effect of a culture supernatant of the human dental pulp stem cells.
[Figure 9] Figure 9 shows a vascular-like structure constructing effect (tube formation) of a culture supernatant of the human dental pulp stem cells.
[Figure 10] Figure 10 shows a vascular endothelial cell recruiting effect of a culture supernatant of the human dental pulp stem cells.
[Figure 11] Figure 11 shows an immunosuppressive effect (ability to suppress the proliferation of CD4-positive T cells) of the human dental pulp stem cells.
[Figure 12] Figure 12 shows a motor function improving effect (A) and nerve regenerative effect (B) of the human dental pulp stem cells on chronic-phase spinal cord injury in a rat spinal cord injury model.
[Figure 13] Figure 13 shows a motor function improving effect of the human dental pulp stem cells on perinatal hypoxic ischemic encephalopathy in a newborn rat hypoxic ischemic encephalopathy model.
[Figure 14] Figure 14 shows an improving effect on the volume of femoral blood flow (A) and an improving effect on limb necrosis (B) in an immunodeficient rat severe lower limb ischemia model.
[Figure 15] Figure 15 shows an amount of BDNF production (A) and an amount of VEGF production (B) of the human dental pulp stem cells.
[Figure 16] Figure 16 shows a vascular endothelial cell proliferative effect of a culture supernatant of the human dental pulp stem cells.
[Figure 17] Figure 17 shows a vascular-like structure constructing effect (tube formation) of a culture supernatant of the human dental pulp stem cells.
[Figure 18] Figure 18 shows amounts of cytokine production of various stem cells.
[Figure 19] Figure 19 shows an increasing effect on a vessel area (A) and an increasing effect on a vessel number (B) in an immunodeficient rat severe lower limb ischemia model.
[Figure 20] Figure 20 shows anti-inflammatory effects of various stem cells under M2 induction conditions (A) and M1 induction conditions (B) of THP-1 cells.
[Figure 21] Figure 21 shows proliferation rates of human dental pulp stem cell culture in various serum-free media and a serum-free medium containing hPL.
[Figure 22] Figure 22 shows a motor function improving effect of the human dental pulp stem cells on severe perinatal hypoxic ischemic encephalopathy in a newborn rat severe perinatal hypoxic ischemic encephalopathy model.

### [Description of Embodiments]

### 1. Stem cell population derived from human dental pulp

The present invention relates to a non-transgenic stem cell population derived from human dental pulp, at least 90% or more of which is CD117-negative, CD73-positive, CD90-positive, and CD105-positive (hereinafter, also referred to as the "stem cell population of the present invention"). Hereinafter, structural or functional characteristics of the stem cell population of the present invention will be described.

### 1.1 Origin

The stem cell population of the present invention is a non-transgenic stem cell population derived from human dental pulp. The "dental pulp" may be any dental pulp of a deciduous tooth or a permanent tooth, and an extracted tooth such as a deciduous tooth or a wisdom tooth is preferred because of its easy availability, etc. It is also desired to use a tooth within 72 hours after tooth extraction, more preferably within 48 hours after tooth extraction. It is particularly desired to use a human deciduous tooth preferably within 48 hours after tooth extraction.

The stem cell population of the present invention may be autologous cells derived from dental pulp of a recipient or may be allogeneic cells derived from dental pulp of another person. The stem cell population is preferably allogeneic cells from the viewpoint of time and cost required for provision and stable quality of the cells.

### 1.2 Marker

"CD117" is a transmembrane protein that functions as tyrosine kinase receptor and is also called c-kit. Previously known dental pulp stem cells are CD117-positive or are a heterogeneous population comprising CD117-positive cells and CD117-negative cells (Hilkens et al., Cell and Tissue Research (2013) 353, 65-78; Deng et al., Frontiers in Cell and Developmental Biology, March 2021, volume 9, Article 661116; Ferro et al., PLoS ONE July 2012, volume 7, Issue 7, e41774; and Lei et al., Stem Cell International, Volume 2021, Article ID 8886854). By contrast, the stem cell population of the present invention is characterized by being CD117-negative. Also, the stem cell population of the present invention is characterized by being positive for mesenchymal stem cell markers CD73, CD90, and CD105.

"CD325", "CD49d", and "CD51" are cell surface proteins that function as adhesion factors and are also called N-cadherin (cadherin 2), integrin α4, and integrin αV, respectively. Previously known dental pulp stem cells are CD325-positive or are a heterologous population comprising CD325-positive cells and CD325-negative cells (Deng et al., Frontiers in Cell and Developmental Biology, March 2021, volume 9, Article 661116; and Madhoun et al., Frontiers in Cell and Developmental Biology, October 2021, volume 9, Article 717624). By contrast, the stem cell population of the present invention is preferably CD325-negative. Previously known dental pulp stem cells are a heterologous population comprising both cells positive and negative for CD51 and CD49d (Lei et al., Stem Cell International, Volume 2021, Article ID 8886854; and Alvarez et al., International Journal of Oral Science (2015), 7, 205-212). By contrast, the stem cell population of the present invention preferably exhibits a CD49d- and/or CD51-positive ratio of 90%, 95%, 97%, 98%, or 99% or more.

The stem cell population of the present invention is preferably negative for an endothelial cell marker CD31 and also negative for hematopoietic stem cell markers such as CD34-negative and CD45-negative. The stem cell population of the present invention has a positive ratio of 90% or more, preferably 95% or more, for CD150, a positive marker of mesenchymal stem cells, and is preferably negative for negative markers CD14 and CD19.

The stem cell population of the present invention is preferably negative for HLA-DR, HLA-DQ, CD40, CD80, and CD86 related to immunogenicity.

The stem cell population of the present invention is preferably positive for adhesion factors CD29 (ITGB1), CD44, and CD166 (ALCAM) and negative for CD106 (VCAM).

The stem cell population of the present invention is CD73-positive, CD90-positive, and CD105-positive, preferably is CD73-positive, CD90-positive, and CD105-positive and is CD117-negative, further preferably is CD73-positive, CD90-positive, and CD105-positive and is CD117-negative and CD325-negative.

The term "positive" for a marker protein or a marker gene, as used herein, means that the stem cell population exhibits a positive ratio of 30% or more, preferably 40% or more, 50% or more, 60% or more, 70% or more, or 80% or more, more preferably 90% or more, for the protein or the gene when the stem cell population at passages 2 to 8 is assayed by an approach known in the art (e.g., detection data from flow cytometry is analyzed with an isotype control). Further preferably, the term "positive" for a marker protein or a marker gene means that the stem cell population at passages 2 to 8 has a positive ratio of 91%, 92%, 93%, 94%, 95%, 96%, 97%, 98%, or 99% or more for the protein or the gene. Particularly preferably, the term "positive" to a marker protein or a marker gene means that the stem cell population at passages 2 to 8 has a positive ratio of 99.1% or more, 99.2% or more, 99.3% or more, 99.4% or more, 99.5% or more, 99.6% or more, 99.7% or more, 99.8% or more, 99.9% or more, or 100% to the protein or the gene. The term "negative" for a marker protein or a marker gene means that the stem cell population has a positive ratio of less than 10%, preferably less than 7%, more preferably less than 6% or less than 5%, further preferably less than 3% or less than 2%, for the protein or the gene when detected in the same manner as above (however, the value of negativity does not fall below 1% when fluorescence intensity of 1% in an isotype control is set as a positive-negative threshold). In this context, the passage of the cells is performed in a 60% confluent state for adhesion culture.

The cell population of the present invention has the following features:
(1) having a CD117-positive ratio of preferably less than 3%, more preferably less than 2%;
(2) having a CD73-positive ratio of preferably 98% or more, more preferably 99% or more;
(3) having a CD90-positive ratio of preferably 98% or more, more preferably 99% or more; and
(4) having a CD105-positive ratio of preferably 98% or more, more preferably 99% or more
   at passages 2 to 8.

The cell population of the present invention may further have, in addition to the features (1) to (4), any two or more, three or more, or four or more of the following features (5) to (20) at passages 2 to 8:
(5) having a CD325-positive ratio of preferably less than 7%, more preferably less than 5%,
(6) having a D49d- and/or CD51-positive ratio of preferably 97% or more, more preferably 98% or more, further preferably 99% or more,
(7) having a CD31-positive ratio of preferably less than 3%, more preferably less than 2%,
(8) having a CD34-positive ratio of preferably less than 7%, more preferably less than 5%,
(9) having a CD45-positive ratio of preferably less than 7%, more preferably less than 5%,
(10) having a CD150-positive ratio of preferably 90% or more, more preferably 95% or more,
(11) having a CD14-positive ratio of preferably less than 7%, more preferably less than 5%,
(12) having a CD19-positive ratio of preferably less than 7%, more preferably less than 5%,
(13) having HLA-DR- and HLA-DQ-positive ratios each of preferably less than 7%, more preferably less than 5%,
(14) having a CD40-positive ratio of preferably less than 7%, more preferably less than 5%,
(15) having a CD80-positive ratio of preferably less than 7%, more preferably less than 5%,
(16) having a CD86-positive ratio of preferably less than 7%, more preferably less than 5%,
(17) having a CD29-positive ratio of preferably 96% or more, more preferably 98% or more,
(18) having a CD44-positive ratio of preferably 96% or more, more preferably 98% or more,
(19) having a CD166-positive ratio of preferably 96% or more, more preferably 98% or more,
(20) having a CD106-positive ratio of preferably less than 7%, more preferably less than 5%.

The detection of a marker protein can be carried out by immunological assay using an antibody, such as ELISA, immunostaining, or flow cytometry. In the case of a protein that is intracellularly expressed without appearing on cell surface, the protein of interest can be detected by expressing a reporter protein together with the protein and detecting the reporter protein. The detection of a marker gene can be carried out by use of a nucleic acid amplification method and/or a nucleic acid detection method, such as RT-PCR, a microarray, or a biochip.

### 1.3 Cytokine production

The stem cell population of the present invention has a high amount of production of a cytokine useful for tissue regeneration and is rich in the cytokines, as compared with conventional dental pulp stem cells prepared using a medium containing FBS. Particularly, the stem cell population of the present invention has the feature that the ratio of the amount of production of a cytokine useful for tissue regeneration to the amount of production of an inflammatory cytokine is high as compared with the conventional dental pulp stem cells.

Examples of the cytokine useful for tissue regeneration can include SCF (stem cell factor), ANGPTL4 (angiopoietin-like 4), BIGH3 (transforming growth factor-beta-induced), brain-derived neurotrophic factor (BDNF), nerve growth factor (NGF), vascular endothelial growth factor (VEGF), stromal cell-derived factor 1 (SDF-1), monocyte chemoattractant protein-1 (MCP-1), and angiopoietin-2. Examples of the inflammatory cytokine can include IL-6 (interleukin-6).

"SCF" (stem cell factor) is a protein that is generated and secreted (in the present specification, collectively referred to as "produced") by mammalian cells, and is known as a nutritional factor that allows hematopoietic stem cells to proliferation in cultured cell experiments. Also, SCF has been reported to act on neurons or vascular endothelial cells expressing its receptor CD117 and thereby promote neuroprotection (Dhandapani et al., J Neurochem. 2005 Oct; 95 (1): 9-19), neurogenesis (Jin et al., J Clin Invest. 2002 Aug; 110 (3): 311-9; and Osada et al., J Neurosurg Spine. 2010 Oct; 13 (4): 516-23), neurite elongation (Hirata et al., Development. 1993 Sep; 119 (1): 49-56), and angiogenesis (Matsui et al., J Biol Chem. 2004 Apr 30; 279 (18): 18600-7; Sun L., Cancer Cell. 2006 Apr; 9 (4): 287-300; and Kim et al., Cardiovasc Res. 2011 Oct 1; 92 (1): 132-40) in cultured cell experiments or animal experiments. These effects are useful in the treatment of not only nerve disease and ischemic disease but various diseases in need of tissue regeneration, such as intestinal disease and bone disease.

"ANGPTL4"(angiopoietin-like 4) is a protein that is generated and secreted by mammalian cells, and has been reported to have an angiogenic effect (Le Jan et al., Am J Pathol. 2003 May; 162 (5): 1521-8; Hermann et al., Clin Immunol. 2005 Apr; 115 (1): 93-101; and Ma et al., Proc Natl Acad Sci U S A. 2010 Aug 10; 107 (32): 14363-8) and an anti-inflammatory effect (Cho et al., JCI Insight. 2019 Aug 22; 4 (16): e125437) in cultured cell experiments or animal experiments. These effects are useful in the treatment of not only ischemic disease, inflammatory disease, and autoimmune disease but various diseases in need of tissue regeneration.

"BIGH3" (transforming growth factor-beta-induced) is a protein that is generated and secreted by mammalian cells, and is also called βig-H3, keratoepithelin, or TGFBI. BIGH3 has been reported to have an angiogenic effect (Aitkenhead et al., Microvasc Res. 2002 Mar; 63 (2): 159-71) and a bone or cartilage degradation suppressive effect (Ruiz et al., Biomaterials. 2020 Jan; 226: 119544) in cultured cell experiments or animal experiments. These effects are considered useful in the treatment of not only ischemic disease and bone or cartilage disease but various diseases in need of tissue regeneration.

"BDNF" (brain-derived neurotrophic factor) is a protein that is generated and secreted by mammalian cells, and is widely distributed in the brain of mammals. BDNF, which is a neurotrophic factor, is known to promote the development and elongation of neurons and participate in the regulation of synaptic functions (Kowianski et al., Cell Mol Neurobiol (2018) 38: 579-593), and has been reported to have a neuroprotective effect (Lau et al., Cell Reports (2015) 12: 1353-1366; and Numakawa et al., Histol Histopathol (2010) 25: 237-258). These effects are considered useful in the treatment of not only neurodegenerative disease but various diseases in need of nerve tissue regeneration.

"VEGF" (vascular endothelial growth factor) is a protein that is generated and secreted by mammalian cells. VEGF is known to induce the cell division, migration, and differentiation of vascular endothelial cells and thereby cause angiogenesis. VEGF is also considered to participate in angiogenesis or neurogenesis at injury sites through the induction of its expression in nerve tissues that have gotten a hypoxic state due to spinal cord injury (Long et al., Chinese Journal of Traumatology 18 (2015) 293-295). In addition, VEGF is also considered to contribute to axonal regeneration through angiogenesis at nerve injury sites in the peripheral nervous system (Saio et al., Int. J. of Mol. Sci. 2021, 22, 11169). These effects are considered useful in the treatment of not only neurodegenerative disease and various diseases in need of nerve tissue regeneration.

"NGF" (nerve growth factor) is a protein that is generated and secreted by mammalian cells, and is known to have a neuroprotective effect in the central nervous system and to participate in the development or survival of neurons in the peripheral nervous system (Keefe et al., Int. J. of Mol. Sci. 2017, 18, 548; and Mariga et al., Neurobiol Dis 2017, January; 97 (Pt B): 73-79). These effects are considered useful in the treatment of not only neurodegenerative disease and various diseases in need of nerve tissue regeneration.

"MCP-1" (monocyte chemoattractant protein-1) is a protein that is generated and secreted by mammalian cells, and is also called C-C motif chemokine 2 (CCL2). MCP-1 is known to have the effects to enhance chemotaxis of monocyte and T lymphocyte and the ability to induce the differentiation of dendritic cells, and to participate in, for example, neurodegenerative disease (Parkinson's disease, Alzheimer's disease, multiple sclerosis, etc.) whose onset is triggered by chronic inflammation (Singh et al., International Immunopharmacology 101 (2021) 107598). Also, MCP-1 has reported to restore the volume of blood flow by improving collateral circulation via arteriogenesis (Ito et al., Circulation Res. (1997), vol. 80, Issue 6, 829-837). Thus, MCP-1 is considered useful in the treatment of inflammatory disease, neurodegenerative disease, and various diseases in need of vascular formation or nerve tissue regeneration.

"Angiopoietin-2" is a protein that is generated and secreted by mammalian cells, and is known to participate in lymphangiogenesis in lymphatic endothelial cells and vascular remodeling in tissues (Akwii et al., Cells 2019, 8, 471). Angiopoietin-2 is considered useful in the treatment of diseases in need of angiogenesis and tissue regeneration.

"IL-6" is a protein that is generated and secreted by mammalian cells, and is known as an inflammatory cytokine that increases immune response and tissue inflammation. Also, IL-6 is a representative factor of a series of inflammatory cytokine groups called SASP (senescence-associated secretory phenotype) produced by senile cells, and is considered responsible for chronic inflammation associated with senescence (Rolt et al., Biogerontology. 2019 Jun; 20 (3): 359-371; and Di et al., PLoS One. 2014 Nov 24; 9 (11): e113572). Thus, it is not preferred that cells administered for the purpose of treating a disease should produce a large amount of IL-6.

Specifically, the stem cell population of the present invention is capable of producing SCF at a weight ratio of at least 1/20 or more, preferably a weight ratio of at least 1/10 or more, more preferably a weight ratio of 1/10 to 1/2, further preferably a weight ratio of 1/10 to 1/3, more preferably a weight ratio of at least 1/5 or more, further preferably a weight ratio of at least 1/3 or more, relative to IL-6 (however, without exceeding the amount of production of IL-6) at passages 2 to 8. The stem cell population of the present invention is capable of producing at least 0.1 ng, 0.13 ng, or 0.15 ng, preferably at least 0.17 ng, more preferably at least 0.20 ng, of SCF in 48 hours of culture per 1 × 10⁶ cells at passages 2 to 8.

As in previously known dental pulp stem cells, when cells themselves to be administered to a subject are CD117-positive, SCF produced by the administered cells are first taken up into the administered cells themselves via CD117 of the self or other administered cells. This reduces its action efficiency on endogenous cells of a recipient on which SCF is supposed to act. The stem cell population of the present invention has high ability to produce SCF and is CD117-negative, and as such, is capable of exerting an excellent tissue regenerating effect when administered to a subject in need of tissue regeneration.

Specifically, the stem cell population of the present invention has a CD117-positive ratio of preferably less than 3%, more preferably less than 2%; and is capable of producing SCF at a weight ratio of at least 1/20 or more, preferably a weight ratio of at least 1/10 or more, more preferably a weight ratio of at least 1/5 or more, further preferably a weight ratio of at least 1/3 or more, relative to IL-6 (however, without exceeding the amount of production of IL-6) at passages 2 to 8.

The stem cell population of the present invention is capable of producing ANGPTL4 at a weight ratio of at least 3 or more, preferably a weight ratio of at least 5 or more, more preferably a weight ratio of 5 to 20, further preferably a weight ratio of 5 to 15, further preferably a weight ratio of at least 10 or more, particularly preferably a weight ratio of at least 20 or more, relative to IL-6 at passages 2 to 8. The stem cell population of the present invention is capable of producing at least 4 ng, 4.5 ng, or 5.0 ng, preferably at least 6.9 ng, more preferably at least 7.0 ng, of ANGPTL4 in 48 hours of culture per 1 × 10⁶ cells at passages 2 to 8.

The stem cell population of the present invention is capable of producing BIGH3 at a weight ratio of 400 or more, preferably a weight ratio of 450 or more, more preferably a weight ratio of 450 to 1500, further preferably a weight ratio of 450 to 1000, relative to IL-6 at passages 2 to 8. The stem cell population of the present invention is capable of producing at least 400 ng, 450 ng, or 500 ng, preferably at least 550 ng, more preferably at least 600 ng, of BIGH3 in 48 hours per 1 × 10⁶ cells at passages 2 to 8.

In this context, the weight ratio of each liquid factor in a culture supernatant of the stem cell population of the present invention is determined by measuring the amount of each liquid factor contained in the culture supernatant after culture.

The stem cell population of the present invention is
a cell population capable of producing SCF at a weight ratio of at least 1/20 or more, preferably a weight ratio of at least 1/10 or more, more preferably a weight ratio of at least 1/5 or more, further preferably a weight ratio of at least 1/3 or more, relative to IL-6 (however, without exceeding the amount of production of IL-6); producing ANGPTL4 at a weight ratio of at least 3 or more, preferably a weight ratio of at least 5 or more, more preferably a weight ratio of at least 10 or more, further preferably a weight ratio of at least 20 or more, relative to IL-6; and producing BIGH3 at a weight ratio of 400 or more, preferably a weight ratio of 450 or more, more preferably a weight ratio of 450 to 1500, further preferably a weight ratio of 450 to 1000, relative to IL-6
at passages 2 to 8.

The stem cell population of the present invention is capable of producing SCF at a weight ratio of at least 1/10 or more, preferably a weight ratio of at least 1/5 or more, more preferably a weight ratio of at least 1/2 or more, further preferably at a weight ratio of at least 1/3 or more, relative to IL-6 (however, without exceeding the weight of production of IL-6) at passages 2 to 8 when cultured in the presence of a human platelet lysate (hPL) in a medium free of other animal-derived components (particularly, FBS). The stem cell population of the present invention is capable of producing SCF at a weight ratio of at least approximately 3 or more, preferably at least approximately 5 or more, further preferably at least approximately 7 or more when cultured in the presence of hPL in a medium free of other animal-derived components, as compared with when cultured in a medium containing FBS.

The stem cell population of the present invention is capable of producing ANGPTL4 at a weight ratio of at least approximately 5 or more, preferably a weight ratio of at least approximately 10 or more, further preferably a weight ratio of at least approximately 20 or more, relative to IL-6 at passages 2 to 8 when cultured in the presence of a human platelet lysate (hPL) in a medium free of other animal-derived components (particularly, FBS). The stem cell population of the present invention is capable of producing ANGPTL4 at a weight ratio of at least approximately 3 or more, preferably at least approximately 5 or more, further preferably at least approximately 7 or more, when cultured in the presence of hPL in a medium free of other animal-derived components, as compared with when cultured in a medium containing FBS.

The stem cell population of the present invention is capable of producing VEGF at a weight ratio of at least approximately 2 or more, preferably a weight ratio of at least approximately 5 or more, further preferably a weight ratio of at least approximately 10 or more, relative to IL-6 at passages 2 to 8 when cultured in the presence of a human platelet lysate (hPL) in a medium free of other animal-derived components (particularly, FBS). The stem cell population of the present invention is capable of producing VEGF at a weight ratio of at least approximately 2 or more, preferably at least approximately 3 or more, further preferably at least approximately 5 or more, when cultured in the presence of hPL in a medium free of other animal-derived components, as compared with when cultured in a medium containing FBS.

The stem cell population of the present invention can reduce the amount of IL-6 production when cultured in the presence of hPL in a medium free of other animal-derived components, as compared with when cultured in a medium containing FBS.

The stem cell population of the present invention has high ability to produce SCF, ANGPTL4, and BIGH3 and a low production ratio of IL-6, and as such, is capable of safely exerting a tissue regenerating effect with low risk of inflammatory response.

### 1.4 Ability to differentiate

The stem cell population of the present invention is multipotent and, as shown in Examples mentioned later, has the ability to differentiate into at least adipocytes, osteoblasts, chondrocytes, and neurons. The differentiation into the cells of interest can be induced as disclosed in Examples of the present specification or in accordance with a method known in the art (e.g., Marion et al., Methods Enzymol. (2006); 420: 339-361; and Wang et al., Molecular Medicine Reports (2016); 14: 5551-5555) by culturing the stem cell population of the present invention in the presence of a differentiation inducing factor appropriate for the cells of interest.

### 1.5 Physiological activity

The stem cell population of the present invention produces a high level of a cytokine useful for tissue regeneration and has, *in vivo* or *ex vivo,* physiological activity (function) such as a neural progenitor cell proliferative effect, a neurite elongating effect, a vascular endothelial cell proliferative effect, a vascular endothelial cell recruiting effect, a vascular-like structure constructing effect, and an immunosuppressive effect.

### 2. Method for producing stem cell population of present invention

The stem cell population of the present invention can be produced by enzymatically isolating cells from the human dental pulp, culturing the cells in a medium free of FBS (fetal bovine serum) in the presence of a human platelet lysate(hPL), and obtaining a colony-forming cell population as the stem cell population.

The "dental pulp" used in the present invention may be any dental pulp of a deciduous tooth or a permanent tooth, and dental pulp of an extracted tooth such as a deciduous tooth or a wisdom tooth is preferred because of its easy availability. For the extracted tooth, it is desired to use a tooth within 72 hours after tooth extraction, more preferably within 48 hours after tooth extraction. It is particularly desired to use a human deciduous tooth preferably within 48 hours after tooth extraction. Examples of the method for enzymatically isolating the cell population from the collected dental pulp include a method of separating cells by treatment with an enzyme containing collagenase, preferably collagenase and neutral protease (e.g., dispase or thermolysin), and centrifuging the cells for isolation. For isolating the cell population from the collected dental pulp, it is preferred to perform treatment with a reagent containing no component derived from mammals and bacteria.

The isolated cells are cultured in the presence of a human platelet lysate (hPL) in a medium free of other animal-derived serum components, particularly, FBS. The "human platelet lysate (hPL)" is obtained by lysing platelet extracted from human blood by a freeze/thaw cycle, and abundantly contains various growth factors and cytokines necessary for cell culture. The amount of the human platelet lysate (hPL) added to the medium or the content thereof in the medium is not particularly limited and is approximately 5 to 20%, preferably 5 to 15%, more preferably 7 to 15%, most preferably approximately 10% for primary culture. The same holds true for subculture.

The term "approximately" as used herein refers to a value that varies within plus and minus 15%, 10%, 8%, 6%, 5%, 4%, 3%, 2%, or 1% base on a reference value. The term "approximately" preferably refers to the range of plus and minus 10%, 5%, or 1% base on a reference value.

The "medium" used in the present invention is not particularly limited as long as the medium is free of other animal-derived unpurified components, particularly, FBS. A serum-free medium is preferably used. The "serum-free medium" as used herein is a medium free of unadjusted or unpurified serum. A medium contaminated with a purified blood-derived component or animal tissue-derived factor (growth factor) is also included in the serum-free medium as long as it does not impair the object of the present invention. Examples of the serum-free medium can include basal media supplemented with no serum: for example, αMEM medium, DMEM medium, BME medium, Eagle MEM medium, BGJb medium, CMRL 1066 medium, Glasgow MEM (GMEM) medium, Improved MEM Zinc Option medium, IMDM medium, Medium 199 medium, F-12 medium, DMEM/F12 medium, IMDM/F12 medium, Ham's medium, RPMI 1640 medium, Fischer's medium, and mixed media thereof; and commercially available serum-free media for mammalian cells: for example, MesenCult-ACF (STEMCELL Technologies Inc.), STEMPRO MSC SFM (Thermo Fischer Scientific Inc.), and UltraCULTURE Serum-free (Lonza).

The "medium" for clinical application preferably contains no mammal-derived component and is particularly preferably animal-free.

The method for producing the stem cell population of the present invention is preferably a method using a human deciduous tooth within 48 hours after tooth extraction and comprising the steps of: i) enzymatically isolating a cell population from collected dental pulp using a reagent containing no component derived from mammals or bacteria; ii) primary-culturing the isolated cell population in the presence of a human platelet lysate (hPL) in a serum-free medium containing no other animal-derived unpurified components (particularly, FBS) to form colonies; and iii) culturing the obtained colonies in the presence of hPL in a serum-free medium containing no other animal-derived unpurified components (particularly, FBS).

The "medium" may be appropriately supplemented with various nutrient sources necessary for the maintenance and proliferation of the cells or each component necessary for the induction of differentiation without impairing the object of the present invention. Examples of the nutrient source can include carbon sources such as glycerol, glucose, fructose, sucrose, lactose, honey, starch, and dextrin, hydrocarbons such as fatty acids, fat or oil, lecithin, and alcohols, nitrogen sources such as ammonium sulfate, ammonium nitrate, ammonium chloride, urea, and sodium nitrate, inorganic salts such as common salt, potassium salt, phosphate, magnesium salt, calcium salt, iron salt, and manganese salt, monopotassium phosphate, dipotassium phosphate, magnesium sulfate, sodium chloride, ferrous sulfate, sodium molybdate, sodium tungstate, manganese sulfate, various vitamins, and amino acids.

The pH of the medium obtained by blending the components described above is in the range of 6.0 to 9.0, preferably 6.5 to 8.5, more preferably 7.0 to 8.0.

The cells isolated from the dental pulp, and the stem cell population of the present invention are adhesion-cultured. A container is not particularly limited as long as the container can be used for cell culture. A flask, a flask for tissue culture, a dish, a petri dish, a dish for tissue culture, a multidish, a microplate, a microwell plate, a multiplate, a multiwell plate, a microslide, a chamber slide, a petri dish, a tube, a tray, a culture bag, and a roller bottle can be used.

The inoculation density of the cells is not particularly limited and is preferably not too high. For example, cells extracted from the dental pulp of one deciduous tooth is inoculated at an area of 25 to 225 cm², preferably 75 to 150 cm². The culture is carried out at 36°C to 38°C, preferably 36.5°C to 37.5°C, under conditions of 1% to 25% O₂ and 1% to 15% CO₂ with the medium replaced.

The primary culture is preferably performed until colonies are formed and grow and detachment is confirmed. After colony detachment is confirmed, the cells are recovered using a filter or the like. The recovered cells are obtained as a highly homogeneous stem cell population without any special separation approach.

The stem cell population may be further subcultured (expansion-cultured), if necessary. The subculture is carried out using the same medium as that for primary culture. The passage is performed when 60% confluency is attained during adhesion culture. Typically, for the primarily cultured cells, the culture is performed for at least 7 days or longer, preferably 9 days or longer, more preferably 9 to 15 days, in order to confirm colony formation, growth, and detachment, whereas the cells thus passaged can be cultured for at least 1 day or longer, preferably 2 days or longer, more preferably 2 to 3 days. The inoculation density of the cells thus passaged is not particularly limited and is preferably not too high. The cells are inoculated at a density of, for example, 1000 to 2000 cells/cm², preferably 1300 to 1500 cells/cm².

The recovered stem cell population or a culture supernatant thereof is desirably subjected to an endotoxin test, a mycoplasma test, a sterilization test, or the like again for safety.

The produced stem cell population may be cryopreserved (e.g., preserved in a deep freezer of 152°C), if necessary, until use. The cryopreservation is performed, for example, by adding an appropriate cryoprotectant to the medium used in the cell culture. Dextran, DMSO, a commercially available cryopreservation solution, or the like can be used as the cryoprotectant.

### 3. Culture supernatant of stem cell population of present invention

The culture supernatant of the stem cell population of the present invention abundantly contains a cytokine useful for tissue regeneration and can be used in itself as a pharmaceutical composition for tissue regeneration, etc.

Specifically, the culture supernatant of the stem cell population of the present invention has at least one of the following characteristics 1) to 3):
1) comprising SCF at a weight ratio of 1/20 or more, preferably 1/10 or more, more preferably 1/10 to 1/2, further preferably 1/10 to 1/3, relative to IL-6 (however, without exceeding the amount of production of IL-6),
2) comprising ANGPTL4 at a weight ratio of 3 or more, preferably 5 or more, more preferably 5 to 20, further preferably 5 to 15, relative to IL-6,
3) comprising BIGH3 at a weight ratio of 400 or more, preferably 450 or more, more preferably 450 to 1500, further preferably 450 to 1000, relative to IL-6.

The culture supernatant of the stem cell population of the present invention particularly preferably has at least one of the following characteristics 1') to 3'):
1') comprising SCF at a weight ratio of 1/20 or more, preferably 1/10 or more, more preferably 1/5 or more, further preferably 1/3 or more, relative to IL-6 (however, without exceeding the amount of production of IL-6),
2') comprising ANGPTL4 at a weight ratio of 3 or more, preferably 5 or more, more preferably 10 or more, further preferably 20 or more, relative to IL-6, and
3') comprising BIGH3 at a weight ratio of 400 or more, preferably 450 or more, more preferably 450 to 1500, further preferably 450 to 1000, relative to IL-6.

The culture supernatant of the present invention can be cryopreserved until clinical use and can be used in the treatment of various diseases in need of tissue regeneration, in the same way as a pharmaceutical composition mentioned later.

### 4. Pharmaceutical composition comprising stem cell population of present invention

The stem cell population of the present invention abundantly produces a cytokine useful for tissue regeneration, such as SCF, has a low production ratio of an inflammatory cytokine, and is CD117-negative. Therefore, the stem cell population of the present invention has an excellent tissue regenerating effect and can be used as a pharmaceutical composition. The stem cell population of the present invention is provided as a cell medicament, if necessary, together with a culture medium or a culture supernatant.

The pharmaceutical composition of the present invention has at least one of the following characteristics 1) to 3):
1) comprising SCF at a weight ratio of 1/20 or more, preferably 1/10 or more, more preferably 1/10 to 1/2, further preferably 1/10 to 1/3, relative to IL-6 (however, without exceeding the amount of production of IL-6),
2) comprising ANGPTL4 at a weight ratio of 3 or more, preferably 5 or more, more preferably 5 to 20, further preferably 5 to 15, relative to IL-6,
3) comprising BIGH3 at a weight ratio of 400 or more, preferably 450 or more, more preferably 450 to 1500, further preferably 450 to 1000, relative to IL-6.

The pharmaceutical composition of the present invention preferably has at least one of the following characteristics 1') to 3'):
1) comprising SCF at a weight ratio of 1/20 or more, preferably 1/10 or more, more preferably 1/5 or more, further preferably 1/3 or more, relative to IL-6,
2) comprising ANGPTL4 at a weight ratio of 3 or more, preferably 5 or more, more preferably 10 or more, further preferably 20 or more, relative to IL-6,3) comprising BIGH3 at a weight ratio of 400 or more, preferably 450 or more, more preferably 450 to 1500, further preferably 450 to 1000, relative to IL-6.

The number of stem cells contained in the pharmaceutical composition of the present invention is appropriately determined depending on a subject or a target disease. A minimum effective amount is practical in consideration of the timing of administration to a subject and time required for culture. In general, the number of cells contained in the pharmaceutical composition is 1 x 10⁵ or more cells or 1 x 10⁶ or more cells, preferably 1 x 10⁷ or more cells, more preferably 1 x 10⁸ or more cells, further preferably 1 x 10⁹ or more cells. The number of doses is not limited to one, and the pharmaceutical composition may be administered two or more times.

The pharmaceutical composition of the present invention is preferably a parenteral preparation, more preferably a parenteral systemic preparation, particularly, an intravenous preparation. Examples of the dosage form suitable for parenteral administration include injections such as solution-type injections, suspension-type injections, emulsion-type injections, and injections which are prepared upon use, and grafts. The preparation for parenteral administration is in the form of an aqueous or nonaqueous isotonic sterile solution or suspension and is formulated into an appropriate unit dosage form, for example, by appropriately combining pharmaceutically acceptable carriers or vehicles, specifically, sterile water or physiological saline, a medium (particularly, a medium for use in the culture of mammalian cells, such as RPMI), a physiological buffer solution such as PBS, a plant oil, an emulsifier, a suspending agent, a surfactant, a stabilizer, an excipient, a vehicle, an antiseptic, and a binder.

The disease to which the pharmaceutical composition of the present invention is applicable is not particularly limited as long as the disease is in need of tissue regeneration. Examples thereof can include spinal cord injury (including traumatic injury, injury caused by surgical operation, and chronic-phase spinal cord injury), ischemic disease (cerebral infarction, limb ischemia including lower limb ischemia, perinatal hypoxic ischemic encephalopathy, ischemic heart disease including myocardial infarction, etc.), inflammatory disease (sepsis, hepatitis, pancreatitis, nephritis, pneumonitis, etc.), autoimmune disease (rheumatism, SLE, type I diabetes mellitus, etc.), intestinal disease (irritable bowel syndrome, ulcerative colitis, Crohn's disease, Hirschsprung's disease and related syndromes, etc.), and the treatment or regeneration of defects in bone.

### 5. Treatment method using stem cell population of the present invention or culture supernatant thereof

The present invention also provides a treatment method comprising administering the stem cell population of the present invention or the culture supernatant thereof to a subject in need. The subject to be treated is not particularly limited as long as the subject is in need of tissue regeneration. Examples thereof can include the above-described spinal cord injury, ischemic disease, inflammatory disease, autoimmune disease, and intestinal disease. The subject may be such a disease in an acute phase, a subacute phase, or a chronic phase.
The subject to be treated by the treatment method of the present invention may be, for example, acute-phase or subacute-phase spinal cord injury, chronic-phase ischemic disease, chronic-phase inflammatory disease, chronic-phase autoimmune disease, chronic-phase intestinal disease, acute-phase or subacute-phase bone defect, or chronic-phase bone defect.

### 6. Dental pulp stem cell bank

A dental pulp stem cell bank can be prepared by preparing dental pulp-derived stem cell populations from a plurality of donors in accordance with the method of the present invention, and cryopreserving the stem cell populations. A method for cryopreserving the cells can be carried out by a method known in the art, as mentioned above.

### [Examples]

Hereinafter, the present invention will be described more specifically with reference to Examples. However, the present invention is not limited by these Examples.

### Example 1: Preparation of dental pulp stem cell

In a sterile environment, dental pulp was scraped out of an extracted tooth (human deciduous tooth) within 48 hours after tooth extraction, chopped, and then treated with Liberase (MNP-S GMP Grade, Roche) added at a final concentration of 0.05 mg/ml with stirring at 37°C for 15 minutes. A reaction supernatant containing cells was recovered, and the remaining dental pulp tissue was further treated with Liberase added at 0.05 mg/ml with stirring at 37°C for 15 minutes. Cells were recovered from the treated dental pulp tissue through a 70 mm strainer, combined with the supernatant recovered first, and centrifuged. The recovered cells were inoculated to a culture container (CellBind T75 flask) using MEMα (Gibco) containing 10% hPL (AventaCell BioMedical Co., Ltd.), and isolated and cultured for 9 days or longer until colonies were formed, grew, and were detached. Medium replacement was performed 2 days and 9 days after inoculation. After colony detachment was confirmed, cells were recovered using TrypLE Select (Gibco) and expansion-cultured using the same medium as above. Hereinafter, human dental pulp stem cells prepared by this method are also referred to as the present human dental pulp stem cells.

### Example 2: Surface marker analysis (1)

Human dental pulp stem cells prepared from eight donors in accordance with Example 1 were reacted at 1 x 10⁵ cells per donor with phycoerythrin (PE)-labeled antibodies against various surface markers (CD117 (#313204), CD31 (#303105), CD34 (#343606), CD45 (#368510), CD90 (#328110), CD105 (#323206), CD73 (#344004), CD325 (#350806), CD49d (#304303), CD51 (#327909), CD29 (#303003), CD44 (#338807), CD166 (#343904), and CD106 (#305805), all manufactured by BioLegend, Inc.), and data was obtained by use of flow cytometry. Analysis was conducted by gating such that an isotype control antibody-positive ratio would be around 1%, and by calculating positive ratios for the various surface markers.

**[Table 1]**

| Positive ratio for surface marker (%) | | | | | | | | |
|---|---|---|---|---|---|---|---|---|
| | Donor 1 | Donor 2 | Donor 3 | Donor 4 | Donor 5 | Donor 6 | Donor 7 | Donor 8 |
| CD117 | 1.99 | 1.82 | 1.36 | 1.36 | 0.58 | 1.10 | 0.91 | 1.20 |
| CD31 | 4.16 | 6.12 | 1.32 | 5.37 | 2.20 | 3.13 | 2.06 | 15.49 |
| CD34 | 1.54 | 1.52 | 0.17 | 0.72 | 6.86 | 19.96 | 1.31 | 3.29 |
| CD45 | 0.97 | 0.92 | 0.22 | 1.36 | 0.46 | 0.90 | 0.56 | 0.51 |
| CD90 | 100.00 | 100.00 | 99.99 | 100.00 | 100.00 | 100.00 | 99.99 | 100.00 |
| CD105 | 100.00 | 99.99 | 100.00 | 100.00 | 100.00 | 100.00 | 100.00 | 100.00 |
| CD73 | 100.00 | 99.99 | 99.99 | 100.00 | 99.99 | 100.00 | 100.00 | 99.99 |
| CD325 | 2.31 | 1.68 | 2.04 | 3.28 | 4.44 | 1.74 | 1.40 | 4.10 |
| CD49d | 96.10 | 99.60 | 99.22 | 99.98 | 99.99 | 99.60 | 99.91 | 99.99 |
| CD51 | 100.00 | 100.00 | 99.99 | 100.00 | 100.00 | 100.00 | 100.00 | 99.99 |

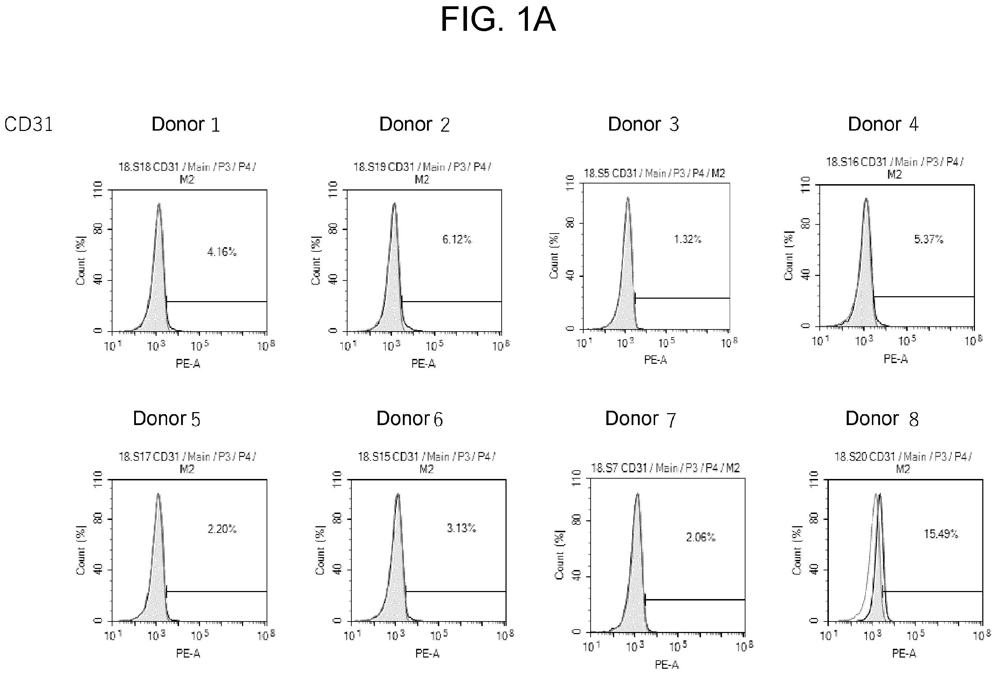

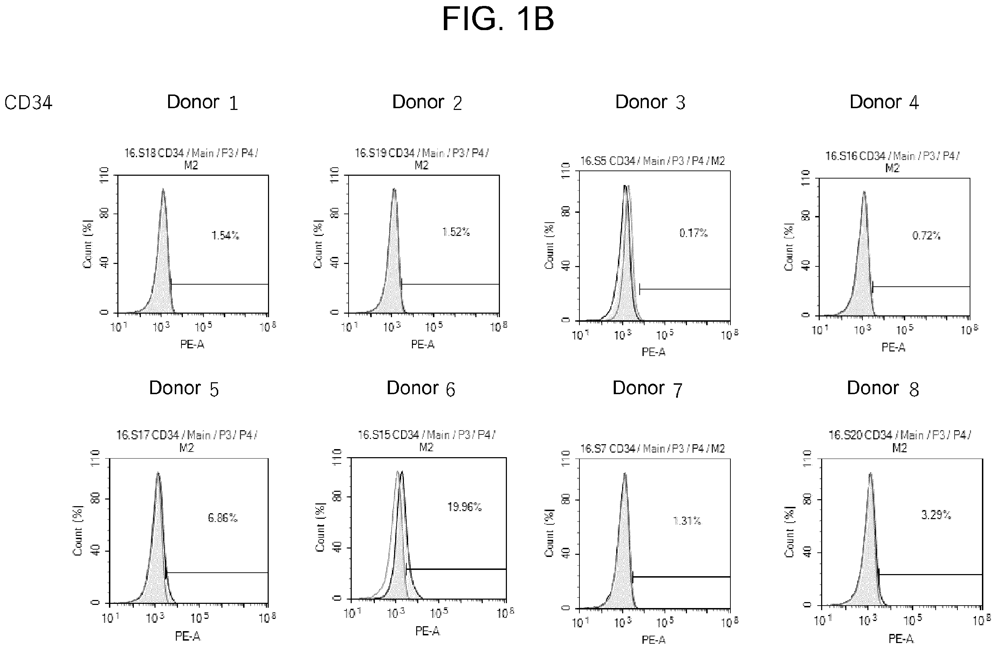

As a result, all the donor cells had a positive ratio of less than 5% for CD117, CD325, and CD45 and were thus determined as being negative therefor. All the donor cells had a positive ratio of 96% or more for CD73, CD90, CD105, CD49d, and CD51 and were thus determined as being positive therefor. The cells of 7 out of the eight donors had a positive ratio of less than 7% for CD31 and CD34 and were thus determined as being negative therefor. The cells of one donor exhibited a CD31-positive ratio of 15.49%, and the cells of another donor exhibited a CD34-positive ratio of 19.96% (Figure 1). All the donor cells had a positive ratio of 99% or more for CD29, CD44, and CD166. A CD106-positive ratio was less than 9% in the cells of 7 out of the eight donors and 10.75% in the cells of the remaining one donor.

### Example 3: Ability to differentiate into adipocyte

The present human dental pulp stem cells obtained in the same manner as in Example 1 were inoculated at 5.76 x 10⁵ cells/2.5 mL/well to a 6-well plate using 10% hPL/MEMa, and cultured until reaching 100% confluency, followed by replacement with an adipose differentiation medium (MEMα, 2% FBS, 500 µM IBMX, 50 µM indomethacin, 5 µg/ml insulin, and 1 µM dexamethasone). After differentiation was induced for 28 days, the cells were stained using a lipid assay kit (Cosmo Bio Co., Ltd., #AK09F), and a fat droplet image was observed under a microscope.

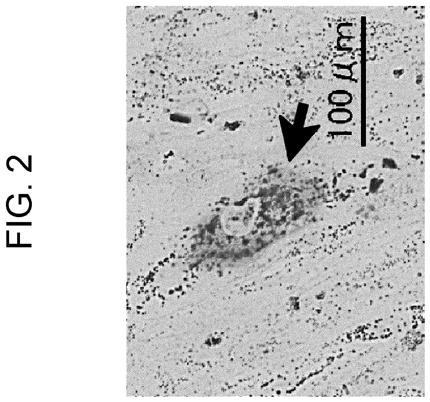

As a result, fat droplets stained red were found in the cells (indicated by the arrow in the drawing), demonstrating that the present human dental pulp stem cells have the ability to differentiate into adipocytes (Figure 2).

### Example 4: Ability to differentiate into osteoblast

The present human dental pulp stem cells obtained in the same manner as in Example 1 were inoculated at 5.76 x 10⁵ cells/2.5 mL/well to a collagen-coated 6-well plate using 10% hPL/MEMa, and cultured until reaching 100% confluency, followed by replacement with a bone differentiation medium (MEMα, 5% FBS, 50 µg of L-ascorbic acid 2-phosphate, 10 nM dexamethasone, and 10 mM β-glycerophosphate). After differentiation was induced for 28 days, the cells were stained using a calcification staining kit (Cosmo Bio Co., Ltd., #AK21), and a calcification image was observed under a microscope.

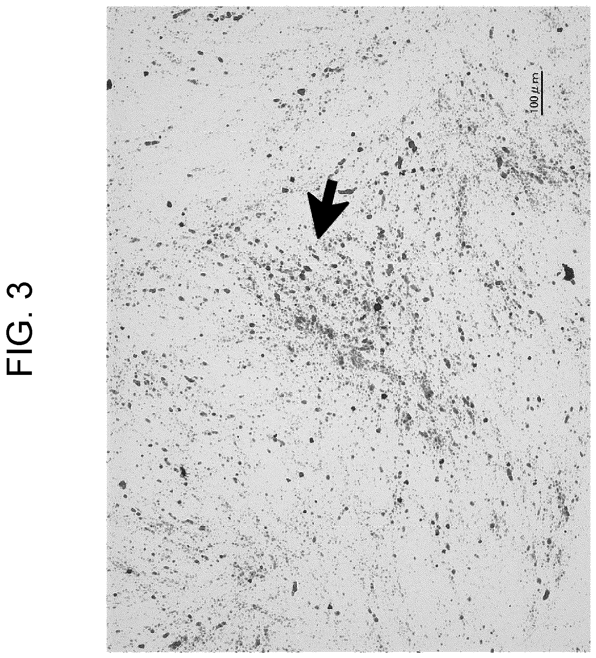

As a result, a calcification image stained red (indicated by the arrow in the drawing) was found, demonstrating that the present human dental pulp stem cells have the ability to differentiate into osteoblasts (Figure 3).

### Example 5: Ability to differentiate into chondrocyte

The present human dental pulp stem cells (donor 1) obtained in Example 1 were inoculated at 2 x 10⁵ cells/200 µL/well to a low-adsorption 96 U-bottom plate (Sumitomo Bakelite Co., Ltd., SUMS9096U) using 10% hPL/MEMa. Spheroid formation was confirmed on the next day, followed by replacement with a cartilage differentiation medium (PromoCell, #C-28012). After differentiation was induced for 21 days, the cells were fixed in 4% paraformaldehyde, embedded in paraffin blocks, and the sliced. The slices were stained using an alcian blue staining solution, and a cartilage tissue image was observed under a microscope.

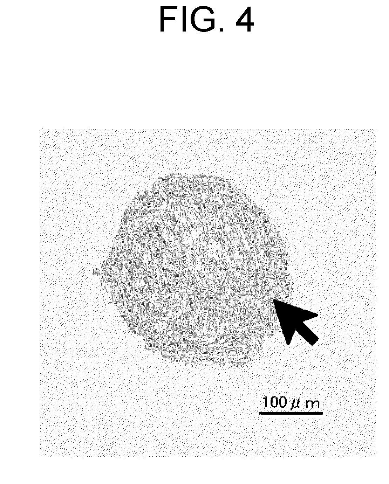

As a result, a cartilage tissue image stained blue (indicated by the arrow in the drawing) was found, demonstrating that the present human dental pulp stem cells have the ability to differentiate into chondrocytes (Figure 4).

### Example 6: Ability to differentiate into neuron

The present human dental pulp stem cells obtained in the same manner as in Example 1 were inoculated at 2.8 x 10³ cells/400 µl/well to poly-D-lysin/laminin cell ware 8-well culture slide (Corning Inc.) using 10% hPL/MEMα, followed by replacement with N2 medium (Neurobasal-A+N2 supplement serum free, 10 ng/ml human EGF, and 10 ng/ml human FGF-basic) 24 hours later. After differentiation was induced for 21 days, the cells were fixed in 4% paraformaldehyde, stained using anti-BIII-tubulin (clone: TU-20) (Millipore, #MAB1637), anti-nestin (clone: 10C2) (Novus Biologicals, LLC, #NB300-266), and anti-neurofilament M (clone: NN18) (Sigma, #5264) antibodies as primary antibodies against neural differentiation markers and Alexa Fluor(R) 488 anti-mouse IgG(H+L) (Jackson ImmunoResearch Laboratories, inc.) as a secondary antibody, and observed under a fluorescence microscope.

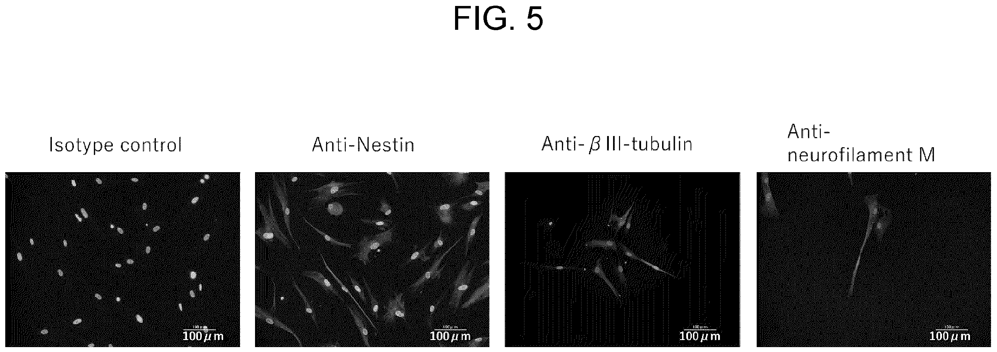

As a result, all the neural differentiation markers were found on the basis of cells immunostained with the antibodies thereagainst, demonstrating that the present human dental pulp stem cells have the ability to differentiate into neurons (Figure 5).

### Example 7: Ability to produce cytokine (1)

The present human dental pulp stem cells were subcultured in 10% hPL/MEMα or 10% FBS/MEMα for 7 days and then inoculated at 5.76 x 10⁵ cells/2.5 ml/well to a 6-well plate using the same medium as above, followed by replacement with hPL-free, FBS-free, and phenol red-free MEMα at 2.5 ml/well on the next day. A culture supernatant was recovered 48 hours later, dispensed, and stored at -80°C. Frozen samples were thawed. SCF, ANGPTL4, BIGH3, and IL-6 concentrations in the samples were measured using Multiplex HGF panel (BioLegend, Inc., #740180), Human ANGPTL4 assay kit (IBL, #27749), Human beta IG-H3 ELISA (Abcam plc, #ab220651), and Multiplex Neuroinflammatory panel (BioLegend, Inc., #740796), respectively, and their respective concentration ratios to IL-6 were calculated (Tables 2 and 3). From the amount of the culture supernatant and the number of inoculated cells, the amounts of various factors contained in the cell culture medium were calculated as the amounts of production per 1 x 10⁶ cells (Tables 4 and 5).

As a result, the culture supernatant of the dental pulp stem cells subcultured in 10% hPL/MEMα had higher concentration ratios to IL-6 and amounts of production per 1 x 10⁶ cells for all of SCF, ANGPTL4, and BIGH3 than those of the culture supernatant of the dental pulp stem cells subcultured in 10% FBS/MEMa.

**[Table 2]**

| Concentrations and ratios of SCF, ANGPTL4, BIGH3, and IL-6 produced by human dental pulp stem cell cultured in 10% hPL/MEMa | | | | | | |
|---|---|---|---|---|---|---|
| | Concentration in culture supernatant (ng/mL) | | | | Ratio to IL-6 | |
| | Donor 1 | | Donor 2 | | Donor 1 | Donor 2 |
| | n=3 | Mean | n=3 | Mean | Mean | Mean |
| SCF | 0.0406 | 0.0395 | 0.0767 | 0.0606 | 0.29 | 0.21 |
| | 0.0396 | | 0.0598 | | | |
| | 0.0383 | | 0.0452 | | | |
| ANGPTL4 | 1.36 | 1.61 | 2.61 | 3.41 | 12 | 12 |
| | 1.68 | | 3.92 | | | |
| | 1.79 | | 3.69 | | | |
| BIGH3 | 71.5 | 128 | 123 | 140 | 944 | 487 |
| | 166 | | 130 | | | |
| | 147 | | 167 | | | |
| IL-6 | 0.167 | 0.136 | 0.266 | 0.288 | | |
| | 0.122 | | 0.261 | | | |
| | 0.119 | | 0.337 | | | |

**[Table 3]**

| Concentrations and ratios of SCF, ANGPTL4, BIGH3, and IL-6 produced by human dental pulp stem cell cultured in 10% FBS/MEMa | | | | | | |
|---|---|---|---|---|---|---|
| | Concentration in culture supernatant (ng/mL) | | | | Ratio to IL-6 | |
| | Donor 1 | | Donor 2 | | Donor 1 | Donor 2 |
| | n=3 | Mean | n=3 | Mean | Mean | Mean |
| SCF | 0.0125 | 0.0117 | 0.0135 | 0.0145 | 0.05 | 0.04 |
| | 0.0112 | | 0.0153 | | | |
| | 0.0114 | | 0.0149 | | | |
| ANGPTL4 | 0.417 | 0.574 | 0.143 | 0.141 | 2.3 | 0.36 |
| | 0.592 | | 0.143 | | | |
| | 0.712 | | 0.138 | | | |
| BIGH3 | 71.2 | 77.2 | 54.2 | 53.5 | 309 | 135 |
| | 58.3 | | 58.3 | | | |
| | 101.9 | | 48.0 | | | |
| IL-6 | 0.350 | 0.249 | 0.375 | 0.397 | | |
| | 0.196 | | 0.486 | | | |
| | 0.202 | | 0.331 | | | |

**[Table 4]**

| Amounts of SCF, ANGPTL4, BIGH3, and IL-6 produced by human dental pulp-derived cell cultured in 10% hPL/MEMa | | | | |
|---|---|---|---|---|
| | Amount of secretion into culture supernatant (ng/10⁶ cells) | | | |
| | Donor 1 | | Donor 2 | |
| | n=3 | Mean | n=3 | Mean |
| SCF | 0.176 | 0.171 | 0.333 | 0.263 |
| | 0.172 | | 0.260 | |
| | 0.166 | | 0.196 | |
| ANGPTL4 | 5.89 | 6.99 | 11.3 | 14.8 |
| | 7.30 | | 17.0 | |
| | 7.78 | | 16.0 | |
| BIGH3 | 311 | 557 | 535 | 609 |
| | 723 | | 564 | |
| | 639 | | 727 | |
| IL-6 | 0.724 | 0.591 | 1.16 | 1.25 |
| | 0.531 | | 1.13 | |
| | 0.516 | | 1.46 | |

**[Table 5]**

| Amounts of SCF, ANGPTL4, BIGH3, and IL-6 produced by human dental pulp-derived cell cultured in 10% FBS/MEMa | | | | |
|---|---|---|---|---|
| | Amount of secretion into culture supernatant (ng/10⁶ cells) | | | |
| | Donor 1 | | Donor 2 | |
| | n=3 | Mean | n=3 | Mean |
| SCF | 0.0542 | 0.0508 | 0.0584 | 0.0631 |
| | 0.0486 | | 0.0663 | |
| | 0.0496 | | 0.0646 | |
| ANGPTL4 | 1.81 | 2.49 | 0.619 | 0.613 |
| | 2.57 | | 0.619 | |
| | 3.09 | | 0.600 | |
| BIGH3 | 309 | 335 | 235 | 232 |
| | 253 | | 253 | |
| | 442 | | 208 | |
| IL-6 | 1.52 | 1.08 | 1.63 | 1.72 |
| | 0.852 | | 2.11 | |
| | 0.877 | | 1.44 | |

### Example 8: Neurite elongating effect

The present human dental pulp stem cells (donor 8) were inoculated at a density of 1000 cells/cm² using 10% hPL/MEMα and cultured for 6 days, followed by medium replacement with MEMα. After culture for another 7 days, a culture supernatant thereof was recovered. This culture supernatant was added to a poly-D lysin (PDL)-coated culture plate (Corning Inc.) so that the plate was coated with components contained in the culture supernatant. IMR-32 cells were inoculated to this plate and induced to differentiate into neurons by the addition of all trans retinal on Days 1 to 4 and Days 7 to 8. On Day 9, neurites and nuclei were stained with Tubulin Tracker(TM) Green (Thermo Fisher Scientific Inc.) and Hoechst 33342 (Dojindo Laboratories), respectively, and photographed, followed by neurite length measurement by image analysis using Autoneurite J.

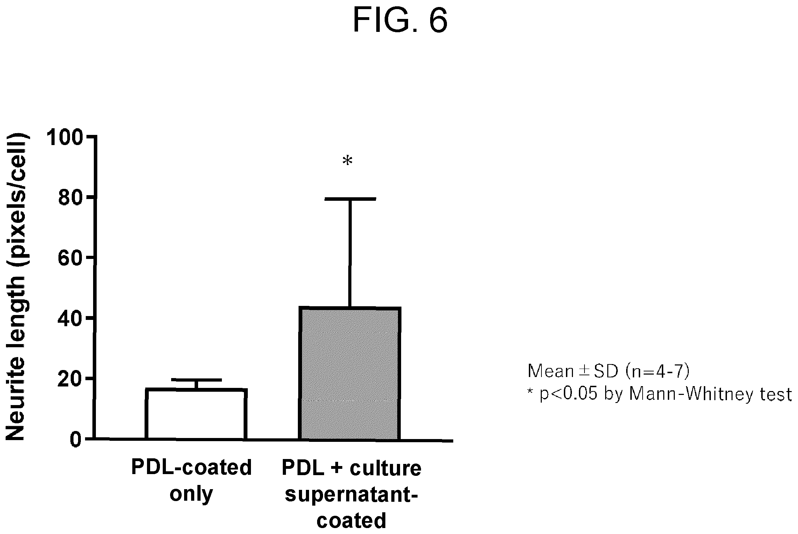

As a result, the lengths of neurites elongating from the IMR-32 cells induced to differentiate into nerve were significantly larger in the plate coated with the culture supernatant than in the plate coated with only PDL. This suggested that neurite elongation was promoted by extracellular matrix or the like secreted by the present human dental pulp stem cells (Figure 6). This effect is important for nerve regeneration.

### Example 9: Neural progenitor cell proliferative effect

Human neural progenitor cells (ENStemA, Millipore) were suspended (5 x 10⁴ cells/100 µl/ well in a 96-well plate) in ENstem expansion medium containing L-glutamine and FGF, and inoculated to Corning BioCoat poly-L-ornithine/laminin-coated multiwell plate. On the next day, the medium was replaced with L-glutamine-containing ENstem expansion medium supplemented with either the dental pulp stem cell culture supernatant recovered in Example 8 or MEMα (control) at a ratio of 20% or 40%, and the cells were further cultured for 3 days. CCK-8 was added to the neural progenitor cells, and the number of neural progenitor cells was evaluated by measuring absorbance at 450 nm 1 hour later.

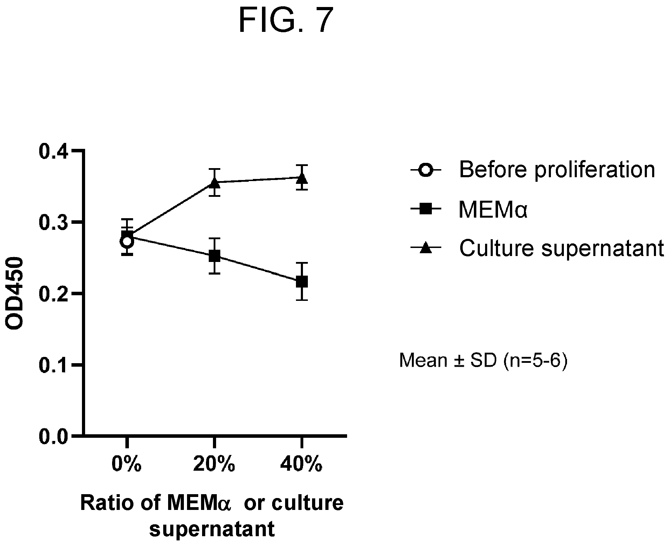

As a result, absorbance was decreased in a manner dependent on the amount of MEMα added in the neural progenitor cells supplemented with MEMα, whereas absorbance was rather elevated in the neural progenitor cells supplemented with the culture supernatant. This suggested that neural progenitor cells proliferated in response to a nutritional factor or the like secreted by the present human dental pulp stem cells (Figure 7). This effect is important for nerve regeneration.

### Example 10: Vascular endothelial cell proliferative effect (1)

HUVEC (human umbilical vein endothelial cell line, Kurabo Industries Ltd.) was suspended in Humedia/2% FBS and then inoculated (2.5 x 10³ cells/well) to a 96-well plate. On the next day, the dental pulp stem cell culture supernatant recovered in Example 8, which was mixed with MEMα at various ratios and further supplemented with 2% (final concentration) FBS, was added in equal amounts from above Humedia/2% FBS in the wells, and the cells were cultured for 3 days. CCK-8 was added to HUVEC and reacted at 37°C for 4 hours. Then, the number of HUVEC cells was evaluated by measuring OD450.

As a result, absorbance was elevated in a manner dependent on the ratio of the culture supernatant. This suggested that vascular endothelial cells proliferated in response to a nutritional factor or the like secreted by the present human dental pulp stem cells (Figure 7). This effect is important for angiogenesis.

### Example 11: Vascular-like structure constructing effect (1)

HUVEC suspended in the dental pulp stem cell culture supernatant recovered in Example 8 or MEMα was inoculated at 1 x 10⁵ cells/well to Extracellular matrix gel (Angiogenesis Assay Kit, Abcam plc) prepared in a 96-well plate, and cultured for 20 hours. The wells were photographed in the bright field, and a branching interval was measured by image analysis using Angiogenesis Analyzer for ImageJ to study a tube forming (vascular-like structure constructing) effect.

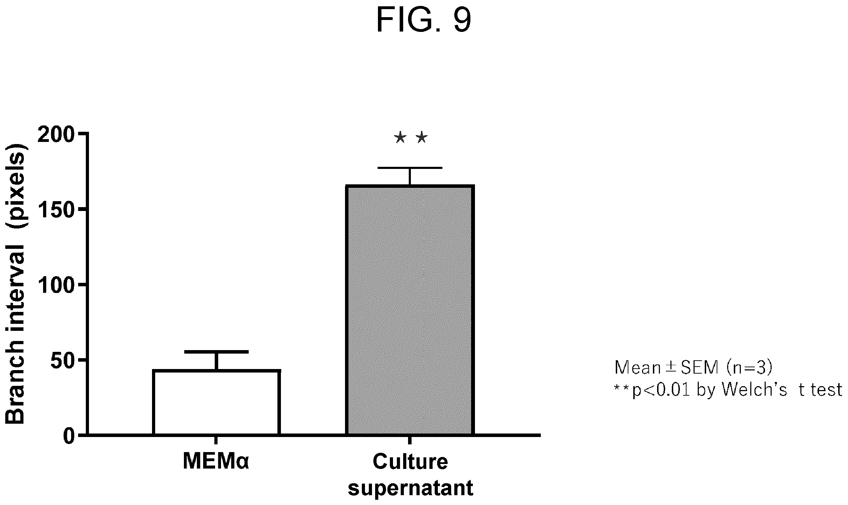

As a result, a branching interval was markedly increased by the addition of the culture supernatant (Figure 9). This suggested that tube formation was promoted by a nutritional factor or the like secreted by the present human dental pulp stem cells. This effect is important for angiogenesis.

### Example 12: Vascular endothelial cell recruiting effect

HUVEC was inoculated (1 x 10⁵ cells/well) to the upper layer of Transwell. The lower layer was filled with the dental pulp stem cell culture supernatant recovered in Example 8 or MEMα, and the cells were cultured for 24 hours. HUVEC moved to the back side (lower layer side) of the upper layer was detached with trypsin. Then, the number of cells thereof was measured as luminescence intensity with CellTiterGlo (Promega Corp.) to evaluate an effect in which HUVEC were recruited to the lower layer.

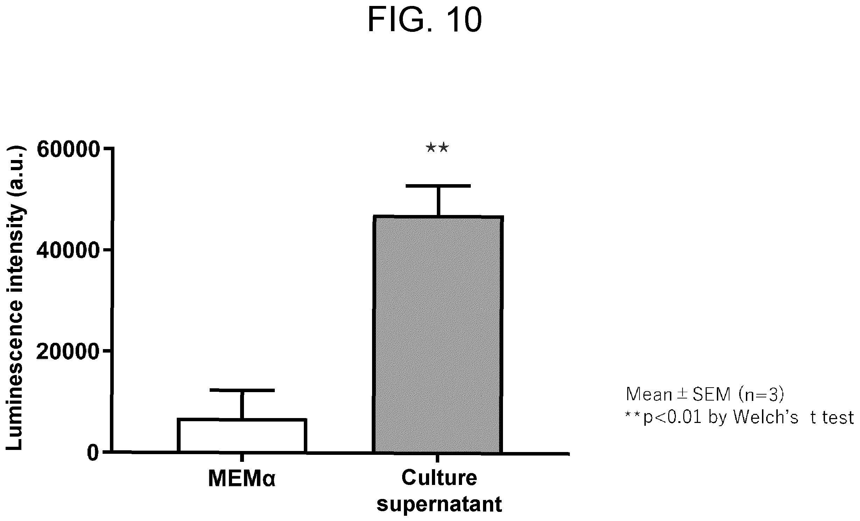

As a result, luminescence intensity was markedly increased by filling the lower layer with the culture supernatant (Figure 10). This suggested that vascular endothelial cells were strongly recruited by a chemokine or the like secreted by the present human dental pulp stem cells. This effect is important for angiogenesis.

### Example 13: Immunosuppressive effect

The present human dental pulp stem cells (1 x 10⁵ cells/well in a 96-well plate) were mixed with CFSE (dye for live cell staining, Thermo Fisher Scientific Inc.)-labeled human PBMC (peripheral blood mononuclear cells, C.T.L). Then, PBMC was stimulated for 6 days by the addition of an anti-CD3 antibody (BioLegend, Inc., #300438) and an anti-CD28 antibody (BioLegend, Inc., #302934) (final concentration: 0.1 µg/ml each). The amount of CFSE is decreased depending on the number of divisions of cells that have taken up CFSE therein. Therefore, the ratio of CFSE^{low} CD4-positive T cells with a signal diluted by proliferation in PBMC was quantified as a CD4-positive T cell proliferation rate by flow cytometry in accordance with a known method (Killer et al., Stem Cell Research & Therapy (2017) 8: 100) to evaluate an immunosuppressive effect of the dental pulp-derived cells.

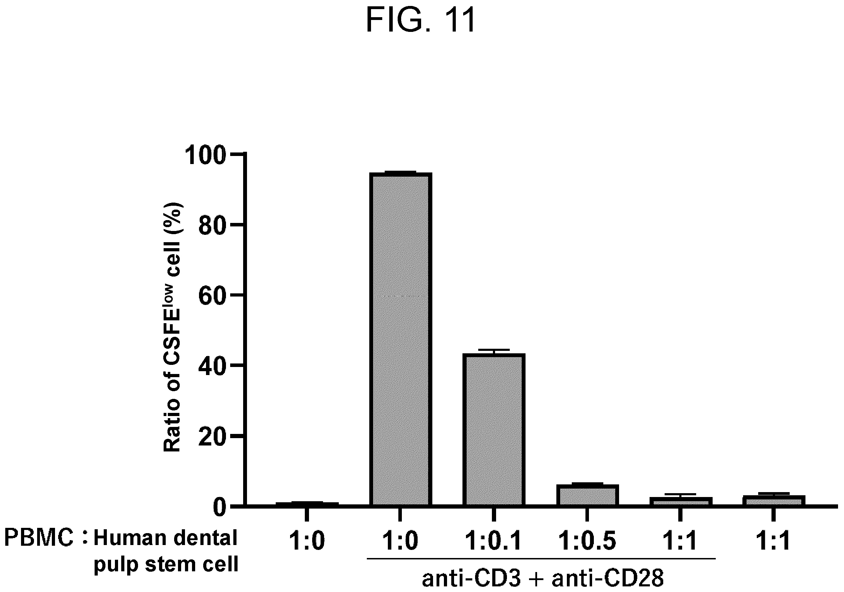

As a result, the proliferation of CD4-positive T cells induced by simulation with an anti-CD3 antibody and an anti-CD28 antibody was markedly suppressed by the present human dental pulp stem cells (Figure 11). This demonstrated that the present cells have a strong immunosuppressive effect.

### Example 14: Effect on spinal cord injury

A weight (diameter: 2.5 mm, 10 g) was dropped from a height of 50 mm to the T9-T10 thoracic spinal cord of a rat (Jcl:SD, SPF, CLEA Japan, Inc.) under anesthesia using MASCIS Impactor (Rutgers University, USA) to prepare a spinal cord injury model. 7, 9, 11, and 13 weeks after model preparation, a solvent or the human dental pulp stem cells of the present invention at 1 x 10⁶ cells into the vein and at 5 x 10⁵ cells into the spinal cavity were administered to study motor function restoring and nerve regenerating effects on chronic-phase spinal cord injury. Specifically, a hindlimb motor function was evaluated over time by the BBB (Basso-Beattie-Bresnahan) test. 15 weeks after model preparation, the rat was fixed by reflux using 4% paraformaldehyde. Then, spinal cord including the injury site was collected, and coronal frozen slices were prepared using OCT compound. The slices were stained with H&E or Luxol Fast Blue (LFB), and stained regions were each quantified by image analysis using Image J to evaluate nerve regeneration. Cyclosporin was intraperitoneally administered at a dose of 10 mg/kg every alternate day from 1 day before initial administration.

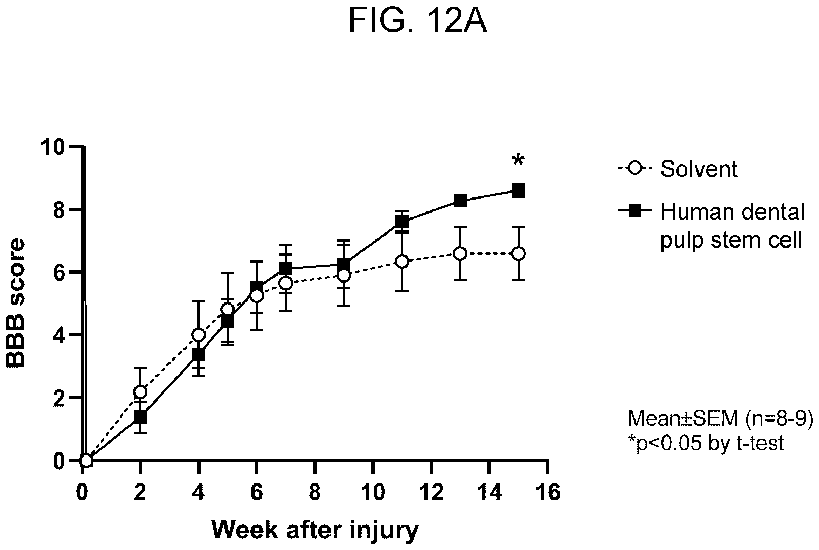

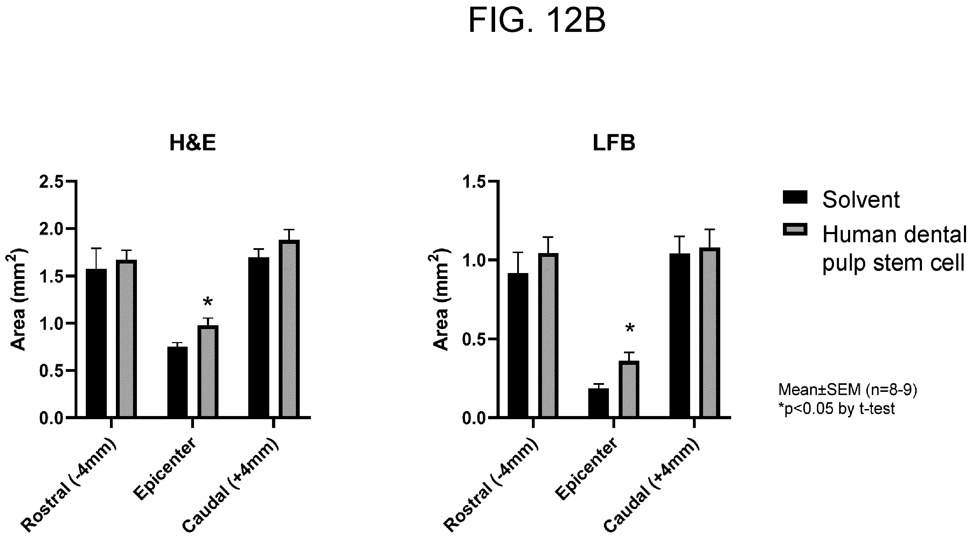

As a result, an upward tendency was found in a BBB score from 4 weeks after initial administration of the cells, and statistically significant elevation was found 2 weeks after final administration (Figure 12A). Both the H&E staining and LFB staining of the spinal cord slices exhibited significant increase in stained area ascribable to the administration of the cells in the epicenter (Figure 12B). This suggested that the present human dental pulp stem cells are capable of regenerating neural parenchyma and myelin sheath and restoring a motor function upon administration using a clinically implementable route and timing in the chronic phase of spinal cord injury.

### Example 15: Effect on perinatal hypoxic ischemic encephalopathy

The left carotid artery of a 7-day-old Wistar/ST rat was ligated under anesthesia, and the rat was left for 1 to 2 hours and then left for 1 hour in a hypoxic (8% O₂) environment to prepare a newborn rat hypoxic ischemic encephalopathy model. On the next day, a solvent or the human dental pulp stem cells of the present invention at 1 x 10⁵ cells were administered into the vein to study a motor function improving effect on perinatal hypoxic ischemic encephalopathy. Specifically, a rotarod test was conducted 41 days after administration, and limb motor coordination and endurance were evaluated by measuring a latent time to fall from a rod.

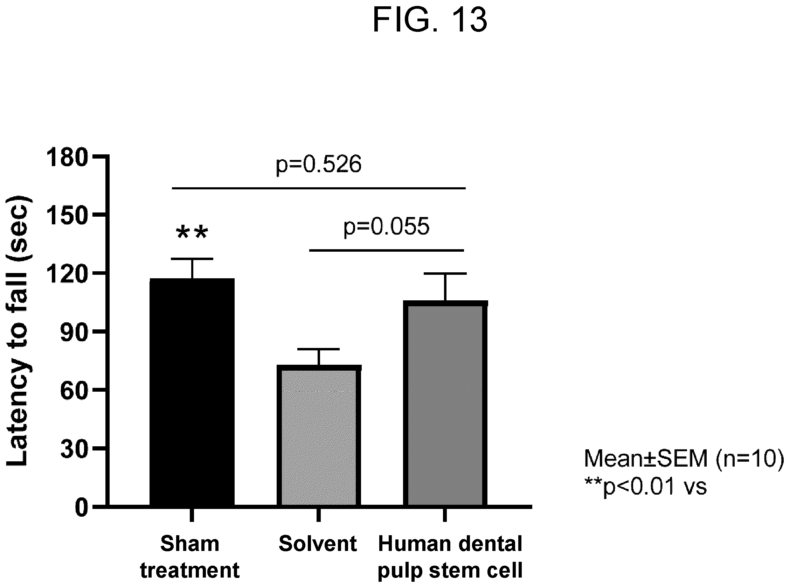

As a result, a drastically shortened latent time to fall, i.e., an obvious disorder in motor function, was found in the solvent administration group compared with a sham treatment group (normal group), whereas the latent time to fall was rarely shortened in the cell administration group, showing a restored motor function (Figure 13). This suggested that the present human dental pulp stem cells are capable of recovering symptoms of cerebral palsy in a relatively short period upon administration using a clinically implementable route and timing in perinatal hypoxic ischemic encephalopathy.

### Example 16: Effect on severe lower limb ischemia

The right common iliac artery and femoral artery and vein of an immunodeficient rat (F344/NJcL/-rnu, CLEA Japan, Inc.) were ligated and cut off to prepare a severe lower limb ischemia model. On the next day, grouping was performed by using the degree of decrease in volume of blood flow compared with normal limbs as an index, and the present human dental pulp stem cells (2 x 10⁶ cells/head) were then intramuscularly administered to ischemic lower limbs to study an effect on severe lower limb ischemia. A blood flow improving effect based on an angiogenic effect was evaluated on the basis of the volume of blood flow compared with normal limbs. An improving effect on limb necrosis was visually evaluated on the basis of the presence or absence of necrosis up to the heel on an individual basis on Day 7 (n = 4 for each group) and Day 14 (n = 6 for each group) after model preparation.

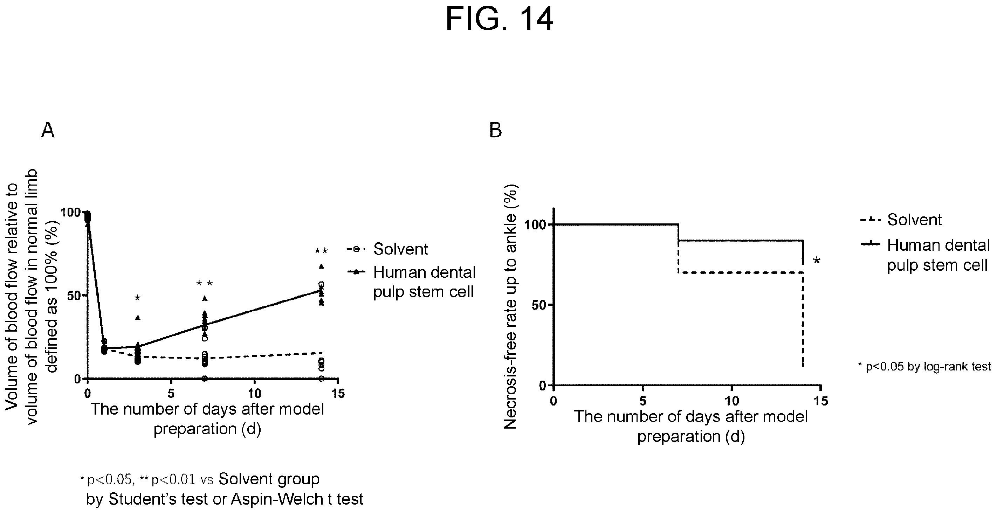

As a result, sustained decrease in volume of blood flow was found over 14 days after ischemia in a solvent administration group, whereas a markedly restored volume of blood flow was found with each passing day in the cell administration group (Figure 14A). The cell administration group also exhibited a high limb necrosis-free rate over 14 days and differed statistically significantly from the solvent administration group (Figure 14B). These results suggested that the present human dental pulp stem cells are capable of restoring blood flow through angiogenesis upon administration using a clinically implementable route and timing in severe lower limb ischemia, and consequently avoiding foot amputation ascribable to limb necrosis.

### Example 17: Ability to produce cytokine (2)

In the same manner as in Example 7, the present human dental pulp stem cells were subcultured in 10% hPL/MEMa or 10% FBS/MEMα and then cultured in a basal medium, and the amount of BDNF production and the amount of VEGF production in the resulting culture supernatant were each measured using LEGENDplex(TM) Human Neuroinflammation panel 1 (BioLegend, Inc., Cat: 740795).

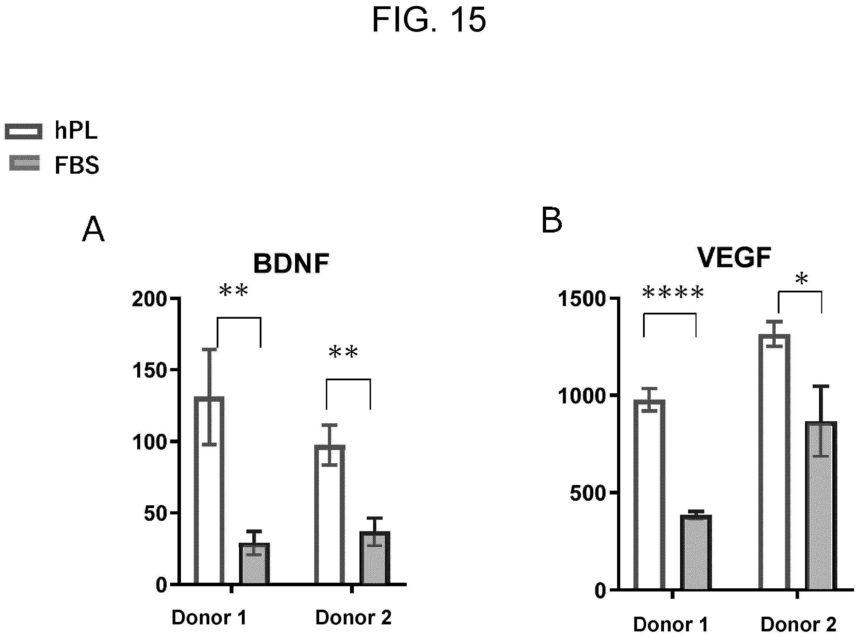

As a result, the culture supernatant after subculture in 10% hPL/MEMa had a higher concentration ratio to IL-6 and amounts of BDNF and VEGF production per 1 x 10⁶ cells than those of the culture supernatant after subculture in 10% FBS/MEMα (Table 6) (Figure 15).

**[Table 6]**

| | 10% hPL/MEMα | | 10% FBS/MEMa | |
|---|---|---|---|---|
| | Ratio to IL-6 | | Ratio to IL-6 | |
| | Donor 1 | Donor 2 | Donor 1 | Donor 2 |
| | Mean | Mean | Mean | Mean |
| BDNF | 0.45 | 0.16 | 0.05 | 0.05 |
| VEGF | 3.34 | 2.19 | 0.68 | 1.1 |

### Example 18: Vascular endothelial cell proliferative effect (2)

In the same manner as in Example 10, the culture supernatant of the present human dental pulp stem cells precultured using 10% hPL/MEMa or 10% FBS/MEMa, which was mixed with MEMα at various ratios and further supplemented with 2% (final concentration) FBS, was added in equal amounts from above Humedia/2% FBS in wells different from those supplemented with the hPL culture supernatant. CCK-8 was added to HUVEC cultured for 3 days after addition of each culture supernatant, and reacted at 37°C for 4 hours. Then, the number of HUVEC cells was evaluated by measuring OD450.

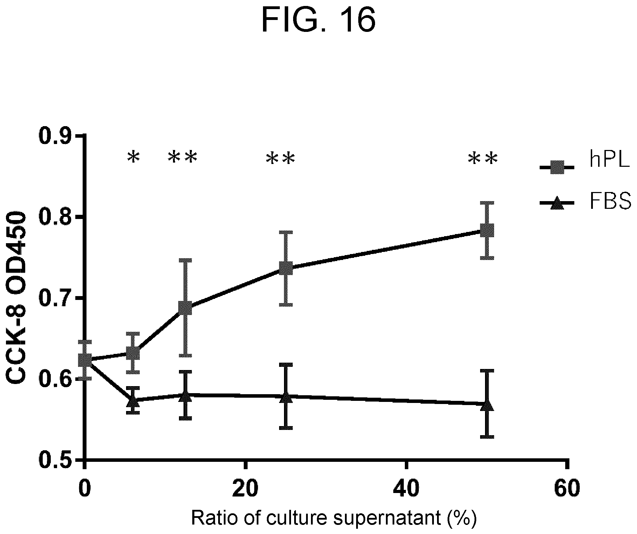

As a result, HUVEC supplemented with the culture supernatant of the cells cultured using 10% FBS/MEMα ("FBS") did not exhibit a proliferative effect as exhibited by HUVEC supplemented with the culture supernatant of the cells cultured using 10% hPL/MEMa ("hPL") (Figure 16).

### Example 19: Vascular-like structure constructing effect (2)

In the same manner as in Example 11, a tube forming effect was studied using HUVEC suspended in the culture supernatant of the present human dental pulp stem cells cultured using 10% FBS/MEMa and HUVEC suspended in the dental pulp stem cell culture supernatant recovered in Example 8.

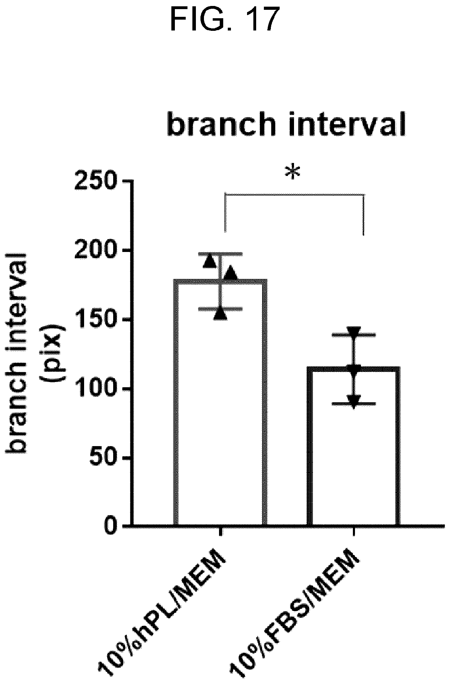

As a result, the dental pulp stem cell culture supernatant recovered in Example 8 ("10% hPL/MEM") was found to promote tube formation as compared with the dental pulp stem cell culture supernatant obtained by culture using FBS ("10% FBS/MEM") (Figure 17).

### Example 20: Comparison of ability to produce cytokine among various stem cells

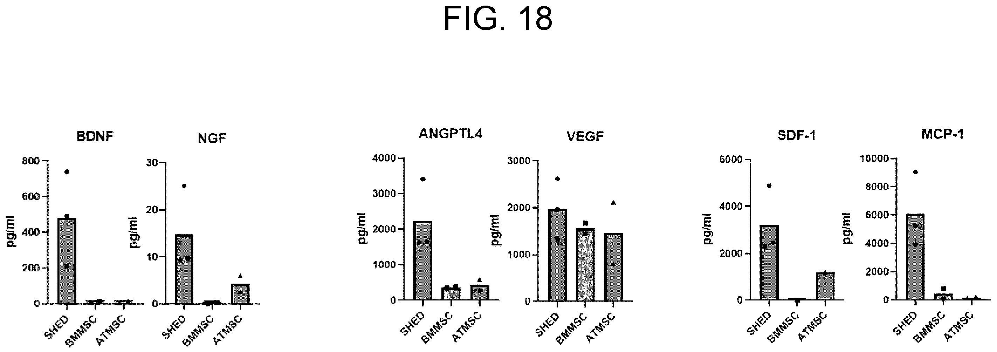

Human deciduous tooth-derived dental pulp stem cells (SHED, the present human dental pulp stem cells), bone marrow-derived stem cells (BMMSC), and adipose tissue-derived stem cells (ATMSC) were inoculated at 5.76 x 10^5 cells/well to a 6-well plate, followed by replacement with Minimum Essential Medium α (MEMα) medium 24 hours after inoculation. The amounts of production of brain-derived neurotrophic factor (BDNF), nerve growth factor (NGF), angiopoietin-like 4(ANGPTL4), vascular endothelial growth factor (VEGF), stromal cell-derived factor 1 (SDF-1), and monocyte chemoattractant protein-1 (MCP-1) were compared among culture supernatants recovered 48 hours thereafter (Figure 18; plots in the drawing respectively show results about cells derived from different donors, and the bar represents a mean thereof).

As a result, the human deciduous tooth-derived dental pulp stem cells were found to have higher ability to produce these liquid factors than that of the other stem cells. It is known that BDNF and NGF are deeply involved in axonal elongation, synaptic transmission, and neuroprotection in the brain nervous system; ANGPTL4 and VEGF are deeply involved in angiogenesis; and SDF1 and MCP-1 were deeply involved as chemokines in cell migration.

### Example 21: Effect on severe lower limb ischemia (2)

For the severe lower limb ischemia model prepared in Example 16, angiogenesis in a dental pulp stem cell administration group and a solvent administration group was quantitatively evaluated by automatic macro with Image J in tissue staining images using an antibody against a smooth muscle cell marker αSMA (ACTA2).

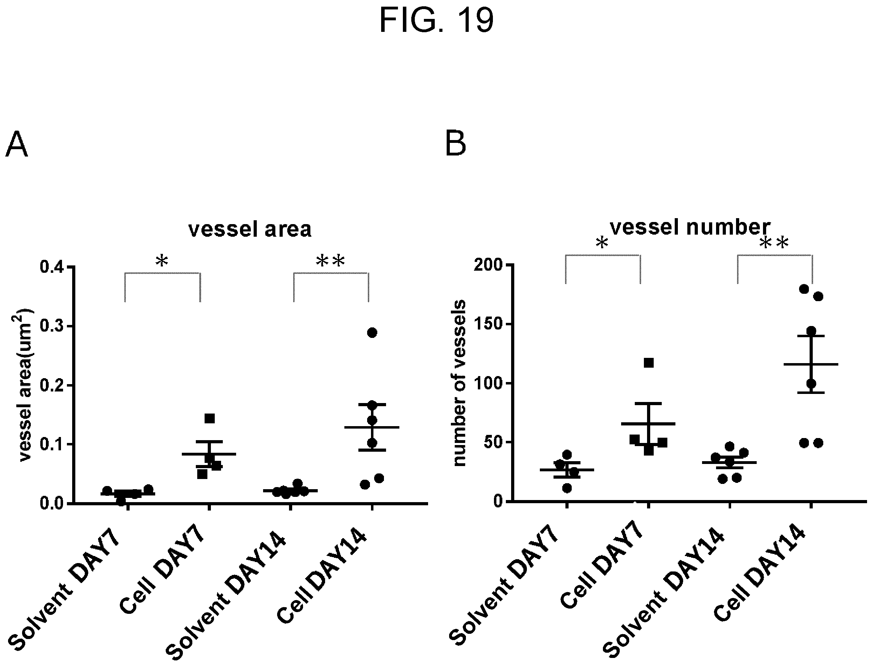

As a result, elevation in both of a vessel area and a vessel number in ischemic lower limb muscle was shown in the cell administration group compared with the solvent administration group (Figure 19). This demonstrated that the dental pulp stem cells are capable of promoting angiogenesis at a severe ischemic site.

### Example 22: Anti-inflammatory effect

A human monocyte leukemia cell line (THP-1) in RPMI1640 (manufactured by Gibco) medium containing 10% FBS and 1 x penicillin-streptomycin (10% FBS/RPMI1640 medium) was stimulated with 10 ng/ml phorbol 12-myristate 13-acetate (PMA) and inoculated at 5 x 10⁵ cells/well to a 24-well plate. Thereon, placed Transwell (Falcon Culture Insert, 24-well, 0.4 µm (HD) PET translucent membrane) having a mesh with a pore size of 0.4 µm, which had been supplemented with Human deciduous tooth-derived dental pulp stem cells (SHED), bone marrow-derived mesenchymal stem cells (BMMSC), adipose tissue-derived mesenchymal stem cells (ATMSC), or permanent tooth-derived dental pulp stem cells (DPSC) at 5 x 10⁴ cells/well (medium: 10% FBS/RPMI1640 medium) respectively , and Culture Insert supplemented with only 10% FBS/RPMI1640 medium was placed on control wells (Cont). Then, 20 ng/ml IL-4 (M2 macrophage induction conditions) or 1 µg/ml LPS and 20 ng/ml IFN-y (M1 macrophage induction conditions) were added thereto. After the resulting cells were cultured for 24 hours, only the THP-1 cells were recovered from each well, and the mRNA levels of transglutaminase-2 (TGM2) and IL-12B were measured by RNA extraction and RT-qPCR.

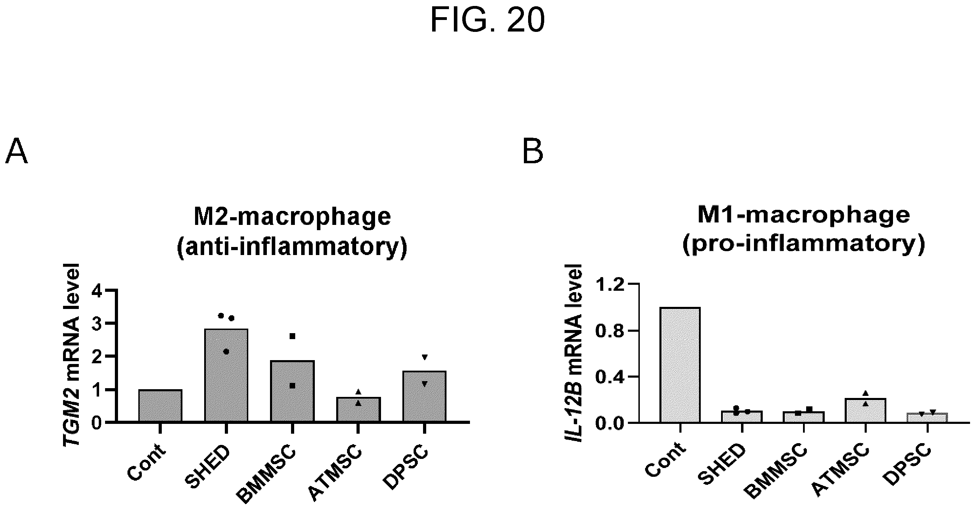

As a result, the transcription of TGM2 in the THP-1 cells stimulated for M2 macrophage induction was significantly promoted by coculture with the human deciduous tooth-derived dental pulp stem cells as compared with coculture with the other stem cells (Figure 20A; plots in the drawing respectively show results about cells derived from different donors, and the bar represents a mean thereof). This demonstrated that the human deciduous tooth-derived dental pulp stem cells are capable of more efficiently promoting the polarization of the THP-1 cells into M2 macrophages having an anti-inflammatory effect than the other stem cells. The transcription of an inflammatory cytokine IL-12B in the THP-1 cells stimulated for M1 macrophage induction was suppressed by coculture with the human deciduous tooth-derived dental pulp stem cells at the same level as in coculture with the other stem cells (Figure 20B; plots in the drawing respectively show results about cells derived from different donors, and the bar represents a mean thereof). This result demonstrated that the human deciduous tooth-derived dental pulp stem cells act to suppress inflammation.

### Example 23: Comparison with animal-free serum-free medium (1) Cell proliferation rate

Human deciduous tooth-derived dental pulp stem cells from four donors were cultured in the same manner as in Example 1. However, culture after cell recovery was performed using 10% hPL/MEMa medium (the medium of the present invention), MesenCult(TM) ACF Plus Medium Kit (manufactured by STEMCELL Technologies Inc.) (serum-free medium 1), or KBM ADSC-4 (manufactured by Kohjin Bio Co., Ltd.) (serum-free medium 2). The inoculation density was set to from 1.3 to 2.6 x 10³ cells/cm², and the cells were inoculated depending on the degree of proliferation so as to attain the same timing of passage.

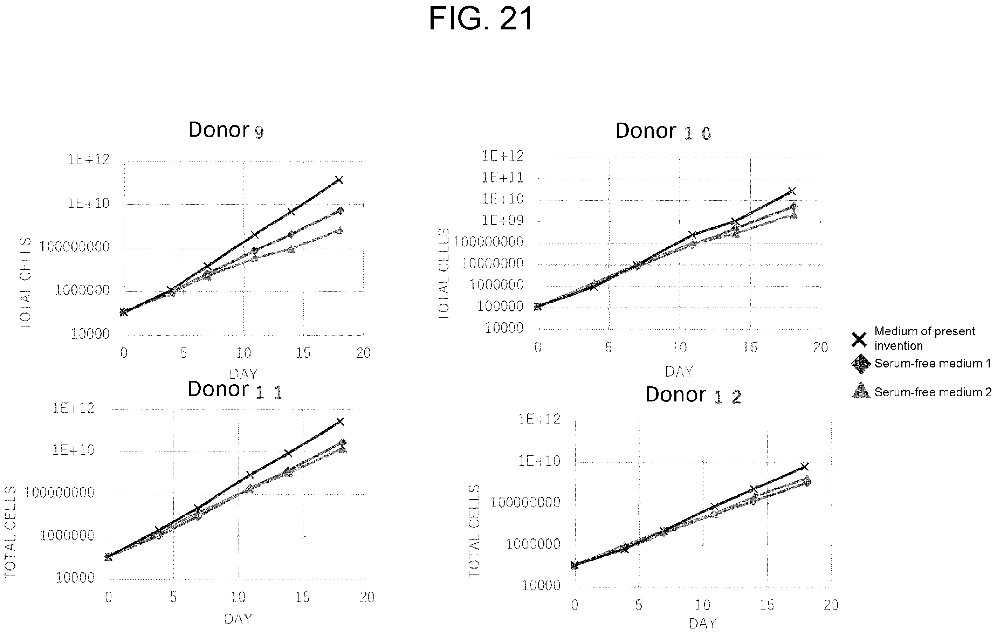

The numbers of cells in each medium at passages 4, 7, 11, 14, and 16 were measured using a cell counter (NucleoCounter NC-202, manufactured by ChemoMetec) and plotted as a proliferation rate (Figure 21).

As a result, the medium of the present invention exhibited a higher cell proliferation rate than that of the other animal-free serum-free media.

### (2) Surface marker analysis

Human deciduous tooth-derived dental pulp stem cells from four donors cultured until passage 8 in the same manner as in (1) were subjected to surface marker analysis in the same manner as in Example 2.

**[Table 7]**

| Donor 9 (positive ratio for surface marker (%)) | | | |
|---|---|---|---|
| | Serum-free medium 1 | Serum-free medium 2 | Medium of present invention |
| CD73 | 99.93 | 99.95 | 99.86 |
| CD105 | 99.80 | 99.55 | 99.41 |
| CD90 | 99.98 | 99.92 | 99.92 |
| CD49d | 97.85 | 99.32 | 99.67 |
| CD51 | 99.92 | 99.90 | 99.94 |
| CD325 | 1.18 | 1.07 | 1.32 |
| CD117 | 1.26 | 0.71 | 1.29 |
| CD31 | 2.66 | 12.21 | 1.79 |
| CD34 | 2.53 | 1.49 | 0.81 |
| CD45 | 1.24 | 1.32 | 0.87 |

**[Table 8]**

| Donor 10 (positive ratio for surface marker (%)) | | | |
|---|---|---|---|
| | Serum-free medium 1 | Serum-free medium 2 | Medium of present invention |
| CD73 | 99.95 | 99.94 | 99.92 |
| CD105 | 99.90 | 98.82 | 98.82 |
| CD90 | 99.90 | 99.97 | 99.93 |
| CD49d | 96.38 | 98.94 | 99.90 |
| CD51 | 99.92 | 99.91 | 99.93 |
| CD325 | 1.34 | 1.10 | 0.87 |
| CD117 | 1.41 | 0.87 | 0.80 |
| CD31 | 8.75 | 14.03 | 2.70 |
| CD34 | 5.47 | 1.82 | 1.31 |
| CD45 | 1.69 | 0.65 | 0.61 |

**[Table 9]**

| Donor 11 (positive ratio for surface marker (%)) | | | |
|---|---|---|---|
| | Serum-free medium 1 | Serum-free medium 2 | Medium of present invention |
| CD73 | 99.93 | 99.91 | 99.93 |
| CD105 | 99.52 | 99.59 | 99.84 |
| CD90 | 99.97 | 99.96 | 99.97 |
| CD49d | 78.77 | 99.49 | 99.88 |
| CD51 | 99.94 | 99.93 | 99.95 |
| CD325 | 1.80 | 1.74 | 3.09 |
| CD117 | 1.32 | 1.24 | 0.58 |
| CD31 | 10.83 | 17.18 | 1.96 |
| CD34 | 3.77 | 2.34 | 3.39 |
| CD45 | 0.55 | 0.08 | 1.39 |

**[Table 10]**

| Donor 12 (positive ratio for surface marker (%)) | | | |
|---|---|---|---|
| | Serum-free medium 1 | Serum-free medium 2 | Medium of present invention |
| CD73 | 99.88 | 99.94 | 99.96 |
| CD105 | 99.67 | 97.96 | 99.39 |
| CD90 | 99.86 | 99.94 | 99.92 |
| CD49d | 59.38 | 97.94 | 99.71 |
| CD51 | 99.97 | 99.92 | 99.92 |
| CD325 | 1.34 | 0.74 | 1.81 |
| CD117 | 1.56 | 0.94 | 0.88 |
| CD31 | 3.13 | 6.03 | 2.28 |
| CD34 | 4.34 | 1.84 | 1.02 |
| CD45 | 1.21 | 0.63 | 0.69 |

### (3) Ability to produce cytokine

Human deciduous tooth-derived dental pulp stem cells from four donors cultured until passage 8 in the same manner as in (1) were evaluated for their ability to produce cytokines in the same manner as in Example 7.

**[Table 11]**

| Amount of cytokine production (pg/ml) | | | | | |
|---|---|---|---|---|---|
| | | Donor 9 | Donor 10 | Donor 11 | Donor 12 |
| IL-6 | Medium of present invention | 29.89833 | 174.11 | 240.8367 | 57.26 |
| | Serum-free medium 1 | 19.76167 | 18.05833 | 28.4933 | 16.56833 |
| | Serum-free medium 2 | 7.72 | 21.22167 | 68.81833 | 3.676667 |
| SCF | Medium of present invention | 25.04333 | 29.095 | 48.93667 | 24.235 |
| | Serum-free medium 1 | 11.08167 | 9.291667 | 14.765 | 10.47167 |
| | Serum-free medium 2 | 9.075 | 7.22 | 12.365 | 7.318333 |
| ANGPTL4 | Medium of present invention | 4029.6574 | 1539.608 | 1510.591 | 2690.014 |
| | Serum-free medium 1 | 0 | 174.6353 | 0 | 0 |
| | Serum-free medium 2 | 661.47817 | 279.3081 | 307.1098 | 499.6569 |
| BDNF | Medium of present invention | 65.87667 | 60.29 | 87.36333 | 95.85 |
| | Serum-free medium 1 | 4.936667 | 3.036667 | 1.996667 | 3.438333 |
| | Serum-free medium 2 | 5.555 | 1.298333 | 5.313333 | 4.29 |
| VEGF | Medium of present invention | 1248.205 | 1057.235 | 491.1667 | 771.9767 |
| | Serum-free medium 1 | 432.4333 | 182.9317 | 122.4117 | 240.2383 |
| | Serum-free medium 2 | 357.3967 | 423.605 | 301.2283 | 198.73 |
| Angiopoietin-2 | Medium of present invention | 43.0566 | 50.79 | 99.16167 | 23.13167 |
| | Serum-free medium 1 | 10.135 | 7.315 | 12.27667 | 20.90833 |
| | Serum-free medium 2 | 5.938333 | 8.49 | 13.38333 | 7.638333 |
| β-NGF | Medium of present invention | 3.212 | 8.419833 | 7.8415 | 3.85 |
| | Serum-free medium 1 | 0.289833 | 0 | 0 | 0 |
| | Serum-free medium 2 | 0 | 0 | 0 | 0 |
| MCP-1 | Medium of present invention | 1980 | 1106.401 | 3604.247 | 3420.026 |
| | Serum-free medium 1 | 131.519 | 279.2285 | 67.07983 | 253.9732 |
| | Serum-free medium 2 | 55.032 | 168.2838 | 112.3287 | 194.4633 |

In the case of culturing human deciduous tooth-derived dental pulp stem cells in the medium of the present invention, the amounts of production of IL-6, SCF, ANGPTL4, BDNF, VEGF, angiopoietin-2, β-NGF, and MCP-1 were found higher than those in the other serum-free media.

The ratios of the amounts of production of SCF, ANGPTL4, BDNF, VEGF, angiopoietin-2, and β-NGF to the amount of production of IL-6 in the medium of the present invention were as shown in Table 12.

**[Table 12]**

| Ratios of amounts of production of SCF, ANGPTL4, BDNF, VEGF, angiopoietin-2, and β-NGF to amount of production of IL-6 | | | | |
|---|---|---|---|---|
| | Donor 9 | Donor 10 | Donor 11 | Donor 12 |
| SCF | 0.84 | 0.17 | 0.20 | 0.42 |
| ANGPTL4 | 134.78 | 8.84 | 6.27 | 46.98 |
| BDNF | 2.20 | 0.34 | 0.36 | 1.67 |
| VEGF | 41.74 | 6.07 | 2.04 | 13.48 |
| Angiopoietin-2 | 1.44 | 0.29 | 0.41 | 0.40 |
| β-NGF | 0.11 | 0.05 | 0.03 | 0.07 |

### Example 24: Surface marker analysis (2)

Human deciduous tooth-derived dental pulp stem cells derived from a single donor were chopped and treated with Liberase in the same manner as in Example 1. Then, the tissue was divided into two parts at the stage of cell recovery through a strainer, and the parts were inoculated into culture containers having 10% hPL/aMEM and 20% FBS/αMEM, respectively, as a medium, and isolated and cultured. A cell population whose colony detachment was confirmed from the culture container having 10% hPL/αMEM medium was expansion-cultured in 10% hPL/αMEM medium, and a cell population whose colony detachment was confirmed from the culture container having 20% FBS/αMEM medium was expansion-cultured in 10% FBS/αMEM medium or 20% FBS/αMEM medium. Each sample was subjected to surface marker analysis by flow cytometry in the same manner as in Example 2 at passage 2. Table 13 shows the surface marker-positive ratios of the cell population expansion-cultured in 10% hPL/αMEM medium ("10% hPL"), the cell population expansion-cultured in 10% FBS/αMEM medium ("10% FBS"), and the cell population expansion-cultured in 20% FBS/αMEM medium ("20% FBS").

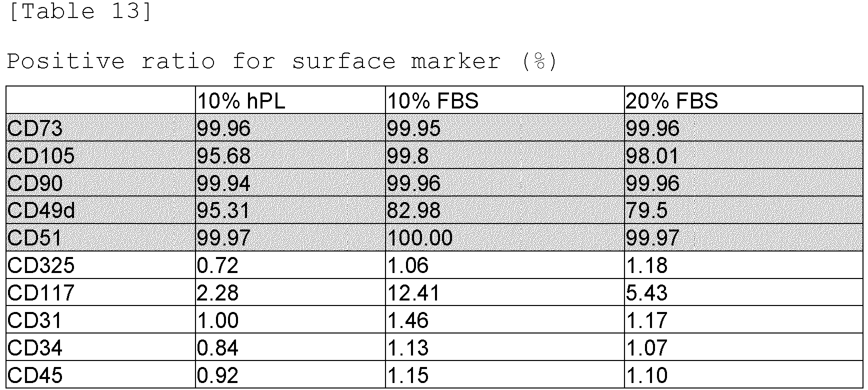

The amounts of production of SCF and ANGPTL4 per 1 x 10⁶ cells after 48 hours of culture in the medium of the present invention were calculated in the same manner as in Example 7 (Table 14).

**[Table 14]**

| SCF and ANGPTL4 (ng/dL) produced by human dental pulp-derived cell cultured in 10% hPL/MEMα | | | | |
|---|---|---|---|---|
| | Donor 9 | Donor 10 | Donor 11 | Donor 12 |
| SCF | 0.11 | 0.13 | 0.21 | 0.11 |
| ANGPTL4 | 17.49 | 6.68 | 6.56 | 11.68 |

### Example 25: Effect on severe perinatal hypoxic ischemic encephalopathy

The right common carotid artery of an SD rat of 7 days after birth was ligated under anesthesia and then cut, and the rat was left for 105 minutes in a hypoxic (8% O₂) environment to prepare a severe hypoxic ischemic encephalopathy model of the newborn rat. At the same time therewith, a sham treatment group was also prepared. At the age of 35 days, grouping was performed by the rotarod method to establish three groups, a sham treatment group, a solvent administration group, and a cell administration group. At the ages of 5 weeks, 7 weeks, 9 weeks, and 11 weeks (four doses at 2-week intervals), the human dental pulp stem cells the present invention was administered (1 × 10⁷ cells/kg) into the tail vein. At the age of 13 weeks, a cylinder test was conducted in a blind manner. At the age of 14 weeks, the animals were euthanized, and the whole brain was excised from half the number of animals in each group (5 animals in the sham treatment group and 6 animals each in the solvent administration group and the cell administration group). Then, tissues were fixed in 4% paraformaldehyde-phosphate buffer solution. Cerebrum tissue samples were embedded in paraffine and sliced in accordance with routine methods, and all the samples were stained with HE and immunostained with myelin basic protein (MBP). Since almost all of the individuals in the solvent administration group and the cell administration group lacked a great part of the cerebrum on the disorder side (right side), the total length of MBP was subjected to image analysis in a blind manner for the left cerebrum tissues on the non-disorder side.

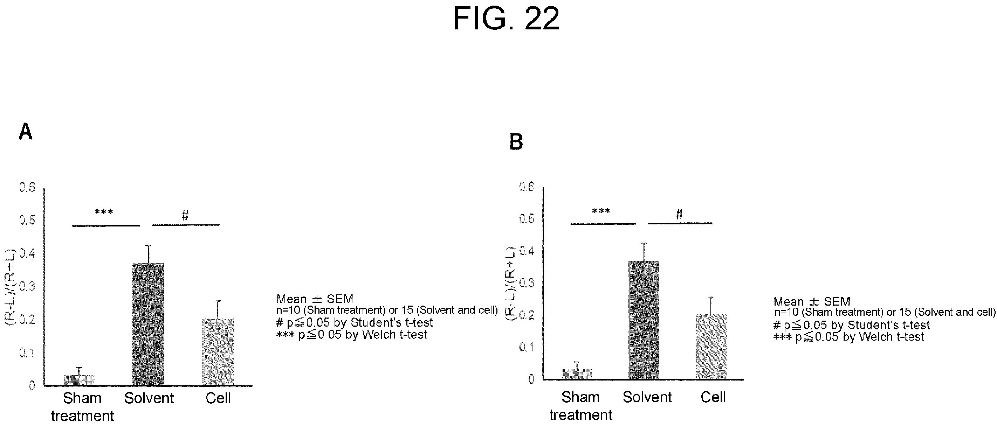

As a result of the cylinder test, the usage rate of the left anterior limb controlled by the brain on the disorder side was significantly high in the cell administration group ("Cell" in the drawing) compared with the solvent administration group ("Solvent" in the drawing). Thus, an improving effect of the administration of the cells on motor dysfunction was found (Figure 22A). Immunostaining with MBP was performed for the purpose of studying the relationship between the administration of the cells and histological change in cerebrum tissue, and a nerve fiber length was calculated from a staining image thereof. Significant increase in total nerve fiber length was found in the cell administration group compared with the solvent administration group (Figure 22B). These results suggest the possibility that under the conditions of this test, the present human dental pulp stem cells elongate (or increase) nerve fiber in the cerebrum on the non-disorder side and contribute to functional restoration of the left anterior limb on the disorder side.

These results demonstrated that repetitive administration using a clinically implementable administration route and administration timing in severe perinatal hypoxic ischemic encephalopathy improves motor dysfunction of cerebral palsy and nerve elongation on the non-disorder side contributes to a partial mechanism of action thereof.

As described above, the human deciduous tooth-derived dental pulp stem cells have high ability to produce cytokines necessary for tissue regeneration such as axonal elongation, synaptic transmission, angiogenesis, and cell migration in the brain nervous system, as compared with other mesenchymal stem cells. On the other hand, the human deciduous tooth-derived dental pulp stem cells have high ability to suppress the induction of inflammatory macrophages and to induce anti-inflammatory macrophages, and therefore act to suppress on inflammation. Hence, the human deciduous tooth-derived dental pulp stem cells are capable of serving as a safer and more highly functional cell medicament.

Useful cytokines such as SCF, ANGPTL4, BDNF, VEGF, angiopoietin-2, β-NGF, and MCP-1 can be highly produced while the production of an inflammatory cytokine (IL-6) is suppressed, by culturing the human deciduous tooth-derived dental pulp stem cells in a serum-free medium containing no FBS in the presence of hPL, as compared with culture in a medium containing FBS.

### [Industrial Applicability]

The dental pulp stem cells of the present invention have multipotency, a neural progenitor cell proliferative effect, a neurite elongating effect, a vascular endothelial cell proliferative effect, a vascular endothelial cell recruiting effect, a vascular-like structure constructing effect, and an immunosuppressive effect and are useful in the treatment of spinal cord injury, ischemic disease, inflammatory disease, neurodegenerative disease, and the like.

All publications, patents, and patent applications cited herein are incorporated herein by reference in their entirety.

## Claims

1. A stem cell population derived from human dental pulp, the stem cell population being **characterized in that** 90% or more of the stem cell population is CD117-negative, CD73-positive, CD90-positive, and CD105-positive, wherein the human dental pulp is human deciduous dental pulp.

2. The stem cell population according to claim 1,
wherein 90% or more of the stem cell population is CD325-negative or is CD51-positive.

3. The stem cell population according to claim 1,
wherein the stem cell population has at least one of the following characteristics 1) to 3):
1) producing SCF (stem cell factor) at a weight ratio of 1/10 or more relative to IL-6,
2) producing ANGPTL4 (angiopoietin-like 4) at a weight ratio of 5 or more relative to IL-6,
3) producing BIGH3 (transforming growth factor-beta-induced) at a weight ratio of 450 or more relative to IL-6.

4. The stem cell population according to claim 1,
wherein the stem cell population has at least one of the following characteristics 1) to 3):
1) producing at least 0.1 ng of SCF (stem cell factor) in 48 hours per 1 × 10⁶ cells,
2) producing at least 500 ng of BIGH3 (transforming growth factor-beta-induced) in 48 hours per 1 × 10⁶ cells,
3) producing at least 5 ng of ANGPTL4 in 48 hours per 1 × 10⁶ cells.

5. The stem cell population according to claim 1,
wherein the stem cell population is obtained by culturing cells enzymatically isolated from the human dental pulp in a medium free of FBS (fetal bovine serum) in the presence of a human platelet lysate.

6. The stem cell population according to claim 5,
wherein the medium is a serum-free medium.

7. A culture supernatant of the stem cell population according to any one of claims 1 to 6, wherein the culture supernatant has at least one of the following characteristics 1) to 3):
1) comprising SCF (stem cell factor) at a weight ratio of 1/10 or more relative to IL-6,
2) comprising ANGPTL4 (angiopoietin-like 4) at a weight ratio of 5 or more relative to IL-6,
3) comprising BIGH3 (transforming growth factor-beta-induced) at a weight ratio of 450 or more relative to IL-6.

8. A pharmaceutical composition comprising the stem cell population according to any one of claims 1 to 6, wherein the stem cell population has at least one of the following characteristics 1) to 3):
1) producing SCF (stem cell factor) at a weight ratio of 1/10 or more relative to IL-6,
2) producing ANGPTL4 (angiopoietin-like 4) at a weight ratio of 5 or more relative to IL-6,
3) producing BIGH3 (transforming growth factor-beta-induced) at a weight ratio of 450 or more relative to IL-6.

9. A pharmaceutical composition comprising a culture supernatant of the stem cell population according to any one of claims 1 to 6, wherein the pharmaceutical composition has at least one of the following characteristics 1) to 3):
1) comprising SCF (stem cell factor) at a weight ratio of 1/10 or more relative to IL-6,
2) comprising ANGPTL4 (angiopoietin-like 4) at a weight ratio of 5 or more relative to IL-6,
3) comprising BIGH3 (transforming growth factor-beta-induced) at a weight ratio of 450 or more relative to IL-6.

10. The pharmaceutical composition according to claim 8 or 9 for treating or preventing any disease selected from spinal cord injury, ischemic disease, inflammatory disease, autoimmune disease, and intestinal disease.

11. A method for producing a stem cell population derived from human dental pulp, the method comprising the steps of:
enzymatically isolating cells from the human dental pulp;
culturing the cells in a medium free of FBS (fetal bovine serum) in the presence of a human platelet lysate; and
obtaining a colony-forming cell population as the stem cell population,
wherein the human dental pulp is human deciduous dental pulp.

12. The method according to claim 11, wherein the medium is a serum-free medium.

13. The method according to claim 11, wherein the medium is animal-free.

14. The method according to any one of claims 11 to 13, wherein 90% or more of the resulting dental pulp stem cell population is CD117-negative.
